# EUROPEAN PATENT APPLICATION

(11) **EP 4 556 573 A2**
(43) Date of publication of application: **21.05.2025**
(21) Application number: 25164517.2
(22) Date of filing: 23.05.2018
(51) Int. Cl.: C12Q 1/6841

(54) **METHODS FOR PERFORMING SPATIAL PROFILING OF BIOLOGICAL MOLECULES**

(30) Priority: 23.05.2017 US 201762509764 P; 23.05.2017 US 201762509765 P; 23.05.2017 US 201762509766 P; 24.05.2017 US 201762510358 P; 24.05.2017 US 201762510356 P; 24.05.2017 US 201762510353 P; 04.10.2017 US 201762568200 P
(62) Divisional of application: 18805393.8
(71) Applicant: Centrillion Technology Holdings Corporation, Grand Cayman, KY1-1104 (KY)
(72) Inventor: ZHOU, Wei, Saratoga, 95070 (US); CHANG, Suzy, Sunnyvale, 94085 (US); POLLOM, T. Scott, Menlo Park, 94025 (US); EDWARDS, Jeremy, Albuquerque, 87106 (US); COSTA, Justin, Union City, 94587 (US); LI, Yaning, Mountain View, 94040 (US); DING, Xun, San Mateo, 94404 (US)
(74) Representative: J A Kemp LLP

(57) **Abstract**

The present disclosure provides methods, devices and systems that enable determination of spatial information of biological molecules by reacting the biological molecules with a zipcode array. In some examples, the zipcode array may code for the spatial positions of biological molecules attached to distinct positions on the zipcode array. In some examples, the spatial positions are 2-dimensional. In some cases, the spatial positions are 3-dimensional. In some examples, the present disclosure provides methods to detect spatial gene expression. In some examples, the present disclosure provides methods to detect spatial distribution of proteins.

## Description

### CROSS-REFERENCE

This application claims the benefit of U.S. Provisional Patent Application No. 62/509,764, filed on May 23, 2017, U.S. Provisional Patent Application No. 62/509,765, filed on May 23, 2017, U.S. Provisional Patent Application No. 62/509,766, filed on May 23, 2017, U.S. Provisional Patent Application No. 62/510,353, filed on May 24, 2017, U.S. Provisional Patent Application No. 62/510,356, filed on May 24, 2017, U.S. Provisional Patent Application No. 62/510,358, filed on May 24, 2017, and U.S. Provisional Patent Application No. 62/568,200, filed on October 4, 2017, each of which is entirely incorporated herein by reference.

### BACKGROUND

Determining the spatial distribution of biological molecules, including, for example, nucleic acids, carbohydrates and proteins, can be of interests in life sciences research, molecular diagnostics, forensic science, personal medicines and other applications. In addition to understanding the gene expression profile of a particular cell or tissue, spatial information of biological molecules within the cell or tissue may also provide other valuable information. For example, spatial distribution of biomolecules in a tissue and a cell may govern many biological processes, ranging from organ development to formation of cell polarity. Advances in elucidating spatial distribution of genes may be used in gene expression profiling of cancer cells when monitoring cancer therapies.

### SUMMARY

While recent advancement in nucleic acid sequencing technologies has greatly improved the routine detection of deoxyribonucleic acid (DNA) sequences, resolving the precise sequences of large biomolecules is still a major challenge. A spatial expression pattern relates to where the gene is expressed, such as what body tissue expresses this gene, which germ layer during development expresses this gene, or which cell type expresses this gene, etc. Methods for studying spatial gene expression are a substantial tool for verifying predicted regulatory interactions and for predicting properties of missing components in a regulation network.

The present disclosure provides methods, devices and systems for the design, manufacturing of spatially encoded nucleic acid arrays which can be used for a variety of molecular detections including detecting mutation distribution within tissue sections.

An aspect of the present disclosure provides a method for detecting spatial distribution of a plurality of target molecules within a biological sample, comprising: (a) providing a substrate comprising a plurality of distinct locations, each of the plurality of distinct locations comprises two or more coordinates; (b) attaching a plurality of zipcodes to the plurality of distinct locations; (c) contacting a biological sample comprising a plurality of target molecules with the substrate, thereby generating a plurality of report molecules, each report molecule comprising: (i) a fragment of a first target molecule of the plurality of target molecules, or a barcode indicating the presence of the first target molecule; and (ii) a first zipcode of the plurality of zipcodes, wherein the first zipcode encodes coordinates of contact on the substrate for the fragment of the first target molecule or the barcode; and (d) sequencing the plurality of report molecules and determining the coordinates of contact, thereby determining the spatial distribution of the plurality of target molecules within the biological sample.

In some embodiments, the plurality of target molecules are deoxyribonucleic acids (DNA), ribonucleic acids (RNA), complementary deoxyribonucleic acids (cDNA), proteins, carbohydrates, lipids, natural products, antigens, metabolites, peptides, aptamer, cells, or binding partners thereof. In some embodiments, the binding partners are antibodies, aptamers, or synthetic antibody mimics. In some embodiments, each binding partner comprises another barcode encoding for the target molecule each binding partner binds to or recognizes. In some embodiments, each zipcode comprises (i) a bottom adapter attached to a distinct location, (ii) a coordinate zipcode attached to the bottom adapter; and (iii) a top adapter attached to the coordinate zipcode. In some embodiments, the coordinate zipcode encodes the two or more coordinates of the distinct location the coordinate zipcode is attached to. In some embodiments, wherein each zipcode comprises (i) a bottom adapter attached to a distinct location, (ii) a lower zipcode attached to the bottom adapter; (iii) a separator sequence attached to the lower zipcode; (iv) an upper zipcode attached to the separator sequence; and (v) a top adapter attached to the upper zipcode. In some embodiments, the lower zipcode encodes a first coordinate and the upper zipcode encodes a second coordinate, and wherein the two or more coordinates comprises the first coordinate and the second coordinate for the distinct location. In some embodiments, the report molecule is deoxyribonucleic acids (DNA) or derivatives thereof. In some embodiments, the biological sample is a tissue section, a derivative of the tissue section, a transfer of the tissue section, or a derivative of the transfer of the tissue section.

Another aspect of the present disclosure provides a method for detecting spatial distribution of a plurality of target molecules in a biological sample, comprising: (a) providing a substrate having a plurality of distinct locations, each distinct location comprising a first and second coordinates; (b) attaching a plurality of zipcodes to each distinct location; thereby encoding the first and second coordinates by the plurality of zipcodes attached to each distinct location; (c) contacting a biological sample comprising a plurality of target molecules with a plurality of binding partners, wherein at least a fraction of the plurality of binding partners bind to or recognize at least a fraction of the plurality of target molecules to form a plurality of first tagged complexes; (d) placing the plurality of first tagged complexes on the substrate, thereby allowing the binding partners in the plurality of first tagged complexes to bind to or recognize at least a fraction of the plurality of zipcodes to form a plurality of second tagged complexes; (e) generating a plurality of report molecules based on the plurality of second tagged complexes; wherein each report molecule encodes for a selected binding partner and a selected zipcode in one of the plurality of second tagged complexes; and (f) sequencing the plurality of report molecules and determining the first and second coordinates and the binding partner for each report molecule; thereby determining the spatial distribution of the plurality of target molecules within the biological sample.

In some embodiments, the plurality of target molecules are deoxyribonucleic acids (DNA), ribonucleic acids (RNA), proteins, complementary deoxyribonucleic acids (cDNA), carbohydrates, lipids, natural products, antigens, metabolites, peptides, aptamers, or cells. In some embodiments, the plurality of binding partners are antibodies, aptamers, or synthetic antibody mimics. In some embodiments, each of the plurality of binding partners comprises a barcode encoding for the target molecule it binds to or recognizes. In some embodiments, each of the plurality of binding partners comprises a barcode encoding for the target molecule it binds to or recognizes. In some embodiments, in (d) the forming the plurality of second tagged complexes is ligating by a ligase, a gap filling, annealing, or hybridizing. In some embodiments, each zipcode comprises (i) a first bottom adapter attached to the distinct location, (ii) a first coordinate zipcode attached to the first bottom adapter; and (iii) a first top adapter attached to the first coordinate zipcode. In some embodiments, the first top adapter is a primer that enables tagging the binding partners. In some embodiments, the first bottom adapter is a sequencing adaptor for sequencing library. In some embodiments, the first coordinate zipcode encodes the first and second coordinates. In some embodiments, each zipcode comprises (i) a first bottom adapter attached to the distinct location, (ii) a first lower zipcode attached to the first bottom adapter; (iii) a first separator sequence attached to the first lower zipcode; (iv) a first upper zipcode attached to the first separator sequence; and (v) a first top adapter attached to the first upper zipcode. In some embodiments, the first lower zipcode encodes the first coordinate and the first upper zipcode encodes the second coordinate. In some embodiments, the first separator sequence comprises a sequence selected from GGG, CCC and TT. In some embodiments, the first lower zipcode comprises from 5 to 24 bases. In some embodiments, the first lower zipcode comprises no more than 16 bases. In some embodiments, the first upper zipcode comprises from 5 to 24 bases. In some embodiments, the first upper zipcode comprises no more than 16 bases. In some embodiments, different zipcodes attached to different distinct locations have an edit distance of 4. In some embodiments, different zipcodes attached to different distinct locations have a long-range minimum edit distance of 5. In some embodiments, the biological sample is a tissue section or a transfer of a tissue section. In some embodiments, the report molecule is a deoxyribonucleic acid (DNA).

Still another aspect of the present disclosure provides a method for detecting spatial distribution of biomolecule expression, comprising: (a) providing a substrate having a plurality of distinct locations, each distinct location comprising a first and second coordinates; (b) attaching a plurality of zipcodes to each distinct location; thereby encoding the first and second coordinates by the zipcodes; (c) contacting a biological sample comprising a plurality of biomolecules with the plurality of zipcodes, thereby attaching at least a fraction of the plurality of zipcodes with at least a fraction of the plurality of biomolecules or fragments thereof, or at least a fraction of copies of the plurality of biomolecules or fragments thereof, and generating a plurality of tagged molecules; and (d) sequencing the plurality of tagged molecules and determining the first and second coordinates for at least the fraction of the plurality of biomolecules; thereby determining the spatial distribution of the plurality of biomolecules within the biological sample.

In some embodiments, the plurality of biomolecules are deoxyribonucleic acid (DNA). In some embodiments, the plurality of biomolecules are complementary deoxyribonucleic acid (cDNA) of ribonucleic acid (RNA). In some embodiments, the RNA is messenger RNA (mRNA). In some embodiments, the method further comprises, prior to (c), reverse transcribing the mRNA to complementary DNA (cDNA). In some embodiments, each zipcode comprises (i) a first bottom adapter attached to the distinct location, (ii) a first coordinate zipcode attached to the first bottom adapter; and (iii) a first top adapter attached to the first coordinate zipcode. In some embodiments, the first top adapter is a primer enabling tagging biomolecules. In some embodiments, the first bottom adapter is a sequencing adaptor for sequencing library. In some embodiments, the first coordinate zipcode encodes the first and second coordinates. In some embodiments, each zipcode comprises (i) a first bottom adapter attached to the distinct location, (ii) a first lower zipcode attached to the first bottom adapter; (iii) a first separator sequence attached to the first lower zipcode; (iv) a first upper zipcode attached to the first separator sequence; and (v) a first top adapter attached to the first upper zipcode. In some embodiments, the first lower zipcode encodes the first coordinate and the first upper zipcode encodes the second coordinate. In some embodiments, the first separator sequence comprises a sequence selected from GGG, CCC and TT. In some embodiments, the first lower zipcode comprises from 5 to 24 bases. In some embodiments, the first lower zipcode comprises no more than 16 bases. In some embodiments, the first upper zipcode comprises from 5 to 24 bases. In some embodiments, the first upper zipcode comprises no more than 16 bases. In some embodiments, different zipcodes attached to different distinct locations have an edit distance of 4. In some embodiments, different zipcodes attached to different distinct locations have a long-range minimum edit distance of 5. In some embodiments, different zipcodes attached to different distinct locations have a long-range minimum edit distance of 5. In some embodiments, the attaching in (c) comprises ligating or annealing.

Another aspect of the present disclosure provides a zip array, comprising: (a) a first location; and (b) a plurality of first zipcodes attached to the first location, wherein each first zipcode comprises (i) a first bottom adapter attached to the first location, (ii) a first lower zipcode attached to the first bottom adapter; (iii) a first separator sequence attached to the first lower zipcode; (iv) a first upper zipcode attached to the first separator sequence; and (v) a first top adapter attached to the first upper zipcode.

In some embodiments, the first location comprises a first coordinate and a second coordinate. In some embodiments, the first lower zipcode encodes the first coordinate, and wherein the first upper zipcode encodes the second coordinate. In some embodiments, the first separator sequence comprises a sequence selected from GGG, CCC and TT. In some embodiments, the first lower zipcode comprises from 5 to 24 bases. In some embodiments, the first lower zipcode comprises no more than 16 bases. In some embodiments, the first upper zipcode comprises from 5 to 24 bases. In some embodiments, the first upper zipcode comprises no more than 16 bases. In some embodiments, the method further comprising, (c) a second location; (d) a plurality of second zipcodes attached to the second location, wherein each second zipcode comprises (i) a second bottom adapter attached to the second location, (ii) a second lower zipcode attached to the second bottom adapter; (iii) a second separator sequence attached to the second lower zipcode; (iv) a second upper zipcode attached to the second separator sequence; and (v) a second top adapter attached to the second upper zipcode. In some embodiments, the second location comprises a third coordinate and a fourth coordinate. In some embodiments, the second lower zipcode encodes the third coordinate and the second upper zipcode encodes the third coordinate. In some embodiments, the second separator sequence comprises a sequence selected from GGG, CCC and TT. In some embodiments, the second lower zipcode comprises from 5 to 24 bases. In some embodiments, the second lower zipcode comprises no more than 16 bases. In some embodiments, the second upper zipcode comprises from 5 to 24 bases. In some embodiments, the second upper zipcode comprises no more than 16 bases. In some embodiments, the first and second locations are adjacent, and wherein both the first and second lower zipcodes pair and the first and second upper zipcodes pair have an edit distance of 4. In some embodiments, the first and second locations are not adjacent, and wherein the first and second lower zipcodes have a long-range minimum edit distance of at least 5. In some embodiments, the first and second locations are not adjacent, and wherein the first and second upper zipcodes have a long-range minimum edit distance of 5. In some embodiments, the first location is no more than 5 µm in length. In some embodiments, the first location is no more than 2 µm in length. In some embodiments, the zipcode array further comprises more than 1 million first locations, wherein each first location is different from another. In some embodiments, the first bottom adaptor is a sequencing adaptor. In some embodiments, the first top adaptor is a primer.

Still another aspect of the present disclosure provides a zipcode array, comprising: (a) a first location; (b) a second location; (c) a plurality of first zipcodes attached to the first location, wherein each first zipcode comprises (i) a first bottom adapter attached to the first location, (ii) a first coordinate zipcode attached to the first bottom adapter; and (iii) a first top adapter attached to the first coordinate zipcode; and (d)a plurality of second zipcodes attached to the second location, wherein each second zipcode comprises (i) a second bottom adapter attached to the first location, (ii) a second coordinate zipcode attached to the second bottom adapter; and (iii) a second top adapter attached to the second coordinate zipcode.

In some embodiments, the first location comprises a first coordinate and a second coordinate. In some embodiments, the first coordinate zipcode encodes the first coordinate and the second coordinate. In some embodiments, the first coordinate zipcode comprises from 6 to 48 bases. In some embodiments, the first coordinate zipcode comprises no more than 32 bases. In some embodiments, the second location comprises a third coordinate and a fourth coordinate. In some embodiments, the second coordinate zipcode encodes the third coordinate and the fourth coordinate. In some embodiments, the second coordinate zipcode comprises from 6 to 48 bases. In some embodiments, the second coordinate zipcode comprises no more than 32 bases. In some embodiments, the first location is no more than 5 µm in length. In some embodiments, the first location is no more than 2 µm in length. In some embodiments, the zipcode array further comprises more than 1 million locations including the first and second locations, wherein each location of the more than 1 million locations is distinguishable from another. In some embodiments, the first bottom adaptor is a sequencing adaptor. In some embodiments, the first top adaptor is a primer.

Another aspect of the present disclosure provides a method for detecting spatial distribution of a plurality of ribonucleic acid molecules in a biological sample, comprising: (a) contacting a first surface comprising a plurality of first oligonucleotides with a biological sample comprising a plurality of ribonucleic acid molecules; (b) extending a fraction of the plurality of first oligonucleotides by a transcriptase using the plurality of ribonucleic acid molecules as templates, thereby generating a plurality of second oligonucleotides, each of the plurality of second oligonucleotides comprising a fragment of complementary DNA (cDNA) of one of the plurality of ribonucleic acid molecules; (c) contacting a zipcode array comprising a plurality of zipcode oligonucleotides with the plurality of second oligonucleotides in the presence of a polymerase, thereby extending the plurality of second oligonucleotides and generating a plurality of third oligonucleotides, each of the plurality of third oligonucleotides comprising one of the plurality of second oligonucleotides and a complementary sequence of one of the plurality of zipcode oligonucleotides; (d) separating the first surface comprising the plurality of third oligonucleotides from the zipcode array; and (e) sequencing the plurality of third oligonucleotides; thereby determining the spatial distribution of the plurality of ribonucleic acid molecules within the biological sample.

In some embodiments, the extending in (c) further comprises a template switching reaction. In some embodiments, the method further comprises, in (b) after transcription, denaturing hybridized second oligonucleotides from ribonucleic acid molecule templates. In some embodiments, the first surface is a gel matrix. In some embodiments, the zipcode array comprises a plurality of distinct locations, and each distinct location comprises a first coordinate and a second coordinate. In some embodiments, a plurality of first zipcode oligonucleotides attached to a first distinct location of the plurality of distinct locations encode the first coordinate of the first distinct location and the second coordinate of the first distinct location. In some embodiments, each first zipcode oligonucleotide comprises (i) a bottom adapter attached to the first distinct location, (ii) a coordinate zipcode attached to the bottom adapter; and (iii) a top adapter attached to the coordinate zipcode. In some embodiments, each first zipcode oligonucleotide comprises (i) a bottom adapter attached to the first distinct location, (ii) a lower zipcode attached to the bottom adapter; (iii) a separator sequence attached to the lower zipcode; (iv) an upper zipcode attached to the separator sequence; and (v) a top adapter attached to the upper zipcode. In some embodiments, the lower zipcode encodes the first coordinate of the first distinct location and the upper zipcode encodes the second coordinate of the first distinct location. In some embodiments, the lower zipcode encodes the first coordinate of the first distinct location and the upper zipcode encodes the second coordinate of the first distinct location. In some embodiments, the biological sample is a tissue section, a derivative of the tissue section, a transfer of the tissue section, or a derivative of the transfer of the tissue section. In some embodiments, at least two second oligonucleotides of the plurality of second oligonucleotides comprise different fragments of complementary DNA (cDNA) sequence(s). In some embodiments, orientation of the plurality of zipcode oligonucleotides on the zipcode array is from 5' to 3'

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "figure" and "FIG." herein), of which:
**FIG. 1** schematically illustrates an example DNA zipcode array chip.
**FIG. 2** schematically shows an example multi-step fabrication method of a DNA zipcode array chip.
**FIG. 3** schematically depicts example embeddings and resulting DNA sequences generated using the methods of the present disclosure.
**FIG. 4** schematically illustrates an example method of calculating edit distance.
**FIG. 5** schematically shows an example method of designing a zipcode array.
**FIG. 6** schematically depicts another example method of designing a zipcode array.
**FIG. 7** schematically illustrates an example of another zipcode array.
**FIG. 8** schematically shows an example distribution of RNA molecules of a mouse brain section determined by methods and devices of the present disclosure.
**FIG. 9** schematically depicts an example method of detecting spatial distribution of a biomolecule.
**FIG. 10** schematically illustrates an example process to analyze RNA sequences from a sample.
**FIG. 11** schematically shows an example process to analyze RNA sequences from a single cell.
**FIG. 12** schematically depicts an example process to analyze single-cell genomics.
**FIG. 13** schematically illustrates an example spatial RNA sequencing analysis results.
**FIG. 14** schematically shows other examples of spatial RNA sequencing
**FIG. 15** schematically depicts still other examples of spatial RNA sequencing analysis.
**FIG. 16** schematically illustrates an example of megabase sequencing.
**FIG. 17** schematically shows an example of how to generating a sequencing library.
**FIG. 18** schematically depicts the steps of capturing a RNA on poly-T oligo-containing gel matrix and template switching reverse transcription to produce a cDNA sequence of the captured RNA.
**FIG. 19** schematically illustrates the steps of denaturing a cDNA sequence synthesized in FIG. 18, and using the cDNA-containing oligo gel matrix to print on a zipcode array chip.
**FIG. 20** schematically shows T cell distribution in a mouse spleen.
**FIG. 21** schematically depicts the spatial analysis of Microglial (Iba1-Tag) in mound brain.
**FIG. 22** schematically illustrates how to assemble a cassette comprising a cover slip, a salinized wafer attached with a casted gel, a microscope slide attached with Jacaranda Chips, and another cover slip.
**FIG. 23** schematically shows on-chip approach for constructing genomic DNA sequencing libraries with a zipcode array.
**FIG. 24** schematically depicts a zipcode printing approach for constructing genomic DNA sequencing libraries.
**FIG. 25** schematically illustrates images of genomic DNA molecules and fragments thereof deposited on a variety of surfaces.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

As used herein, the singular form "a", "an", and "the" include plural references unless the context clearly dictates otherwise.

As used herein, the term "about" generally refers to the indicated numerical value ±10%.

As used herein, open terms, for example, "comprise", "contain", "include", "including", "have", "having" and the like refer to comprising unless otherwise indicates.

As used herein, the term "embedding" and "a string of synthetic steps" generally refer to a series of active and inactive steps designed for forming an individual polymer on the substrate and can be used interchangeably. For example, in cases where light-directed synthetic methods are employed, the "embedding" refer to a series exposure and non-exposure steps.

As used herein, the term "edit distance" generally refers to the minimum number of changes (such as insertions, deletions, substitutions and translocations) needed to convert one polymer into another. For example, the edit distance between sequences AGCGCTTAGCCTAGAGCTCTAG and GCGCTTAGCTTAGAGCTCTATTG is 4.

As used herein, the term "polymer" generally refers to any kind of natural or non-natural large molecules, composed of multiple subunits. Polymers may comprise homopolymers, which contain a single type of repeating subunits, and copolymers, which contain a mixture of repeating subunits. In some cases, polymers are biological polymers that are composed of a variety of different but structurally related subunits, for example, polynucleotides such as DNA composed of a plurality of nucleotide subunits.

As used herein, the term "substrate" generally refers to a substance, structure, surface, material, means, or composition, which comprises a nonbiological, synthetic, nonliving, planar, spherical or flat surface. The substrate may include, for example and without limitation, semiconductors, synthetic metals, synthetic semiconductors, insulators and dopants; metals, alloys, elements, compounds and minerals; synthetic, cleaved, etched, lithographed, printed, machined and microfabricated slides, devices, structures and surfaces; industrial polymers, plastics, membranes; silicon, silicates, glass, metals and ceramics; wood, paper, cardboard, cotton, wool, cloth, woven and nonwoven fibers, materials and fabrics; nanostructures and microstructures. The substrate may comprises an immobilization matrix such as but not limited to, insolubilized substance, solid phase, surface, layer, coating, woven or nonwoven fiber, matrix, crystal, membrane, insoluble polymer, plastic, glass, biological or biocompatible or bioerodible or biodegradable polymer or matrix, microparticle or nanoparticle. Other example may include, for example and without limitation, monolayers, bilayers, commercial membranes, resins, matrices, fibers, separation media, chromatography supports, polymers, plastics, glass, mica, gold, beads, microspheres, nanospheres, silicon, gallium arsenide, organic and inorganic metals, semiconductors, insulators, microstructures and nanostructures. Microstructures and nanostructures may include, without limitation, microminiaturized, nanometer-scale and supramolecular probes, tips, bars, pegs, plugs, rods, sleeves, wires, filaments, and tubes.

As used herein, the term "biological sample" generally refers to any sample containing biological material(s) or molecule(s), or any sample containing derivatives of the biological material(s) or molecule(s). Examples of or sources of biological samples may include any primary, intermediate or semi-processed, or processed samples, e.g., blood, serum, plasma, urine, saliva, spinal fluid, cerebrospinal fluid, milk, or any other biological fluid, skin cells, cell or tissue samples, biopsied cells or tissue, sputum, mucus, hair, stool, semen, buccal samples, nasal swab samples, or homogenized animal or plant tissues as well as cells, bacteria, virus, yeast, and mycoplasma, optionally isolated or purified, cell lysate, nuclear extract, nucleic acid extract, protein extract, cytoplasmic extract, etc. Biological samples can also include, e.g., environmental samples or food samples, to be tested for microorganisms. Examples of biological samples may also include any composition or material containing biomolecule(s), either naturally existing or synthesized, e.g., DNA, RNA, nucleic acid, polynucleotide, oligonucleotide, amino acid, peptide, polypeptide, biological analytes, drugs, therapeutic agents, hormones, cytokines, etc. The biological samples can be provided fresh, such as blood samples obtained from a finger stick or a heel stick and directly applied to a sample node. The biological samples can be provided in a container or via a carrier. In some cases, a biological sample is pretreated or partially treated, e.g., with a lysing agent, such as a detergent (e.g., SDS or Sarcosyl), a precipitating agent, such as perchloric acid, a chaotrope, such as guanidinium chloride, a precipitating agent, such as acetone or an alcohol, or some other agent. In some cases, a biological sample is absorbed to, or stored or maintained in a sample holder, e.g., dry storage of a biological sample in a sample holder.

As used herein, the term "subunit" generally refers to a subdivision of a larger molecule or a single molecule that assembles (or "coassembles") with other molecules to form a larger molecular complex such as polymers. Non-limiting example of subunits include monomers, simple carbohydrates or monosaccharide moieties, fatty acids, amino Acids, and nucleotides.

As used herein, the term "nucleic acid" generally refers to a polymer comprising one or more nucleic acid subunits or nucleotides. A nucleic acid may include one or more subunits selected from adenosine (A), cytosine (C), guanine (G), thymine (T) and uracil (U), or variants thereof. A nucleotide can include A, C, G, T or U, or variants thereof. A nucleotide can include any subunit that can be incorporated into a growing nucleic acid strand. Such subunit can be an A, C, G, T, or U, or any other subunit that is specific to one or more complementary A, C, G, T or U, or complementary to a purine (i.e., A or G, or variant thereof) or a pyrimidine (i.e., C, T or U, or variant thereof). A subunit can enable individual nucleic acid bases or groups of bases (e.g., AA, TA, AT, GC, CG, CT, TC, GT, TG, AC, CA, or uracil-counterparts thereof) to be resolved. In some examples, a nucleic acid is deoxyribonucleic acid (DNA) or ribonucleic acid (RNA), or derivatives thereof. A nucleic acid may be single-stranded or double-stranded.

As used herein, the term "adjacent" or "adjacent to," includes "next to," "adjoining," and "abutting." In one example, a first location is adjacent to a second location when the first location is in direct contact and shares a common border with the second location and there is no space between the two locations. In some cases, the adjacent is not diagonally adjacent.

As used herein, the term "biomolecule" generally refers to any molecule that is present in living organisms or derivative thereof. Biomolecules include proteins, antibodies, peptides, enzymes, carbohydrates, lipids, nucleic acids, oligonucleotides, aptamer, primary metabolites, secondary metabolites, and natural products.

The term "nucleotide," as used herein, generally refers a molecule that can serve as the monomer, or subunit, of a nucleic acid, such as deoxyribonucleic acid (DNA) or ribonucleic acid RNA). A nucleotide can be a deoxynucleotide triphosphate (dNTP) or an analog thereof, e.g., a molecule having a plurality of phosphates in a phosphate chain, such as 2, 3, 4, 5, 6, 7, 8, 9, or 10 phosphates. A nucleotide can generally include adenosine (A), cytosine (C), guanine (G), thymine (T) and uracil (U), or variants thereof. A nucleotide can include any subunit that can be incorporated into a growing nucleic acid strand. Such subunit can be an A, C, G, T, or U, or any other subunit that is specific to one or more complementary A, C, G, T or U, or complementary to a purine (i.e., A or G, or variant thereof) or a pyrimidine (i.e., C, T or U, or variant thereof). A subunit can enable individual nucleic acid bases or groups of bases (e.g., AA, TA, AT, GC, CG, CT, TC, GT, TG, AC, CA, or uracil-counterparts thereof) to be resolved. A nucleotide may be labeled or unlabeled. A labeled nucleotide may yield a detectable signal, such as an optical, electrostatic or electrochemical signal.

As used herein, the term "zipcode" generally refers to a known, determinable, and/or decodable sequence, such as, for example, a nucleic acid sequence (DNA sequence or RNA sequence), a protein sequence, and a polymer sequence (including synthetic polymers, carbohydrates, lipids, etc.), that allows the identification of a specific location of the sequence, e.g., the nucleic acid, in one, two or multiple dimensional spaces. A zipcode can encode the decodable sequence's own location. For example, each of the zipcode may be a nucleic acid (may be many copies in a spatially defined location such as a square feature of any size from about 10 nm to about 1 cm, including for example, no larger than 0.1 µm, no larger than 0.2 µm, no larger than 0.5µm, no larger than 1 µm, no larger than 2 µm, no larger than 5 µm, no larger than 10 µm, no larger than 20 µm, no larger than 30 µm, no larger than 40 µm, no larger than 50 µm, no larger than 100 µm, no larger than 200 µm, no larger than 500 µm, no larger than 1mm, no larger than 2 mm, and no larger than 5 mm. Zipcode arrays can be used to detect the distribution of ribonucleic acid (RNA), protein, deoxyribonucleic acid (DNA) or other molecules distribution in two or three dimensional space. These biomolecules can be detected in tissue, cell, organism or non-living systems. If a nucleic acid sequence is a zipcode, the complementary sequence of the nucleic acid sequence can also be a zipcode. In this disclosure, a zipcode and its complementary copy can encode the same position/location on the zipcode array.

The zipcodes can be designed for precision sequence performance, e.g., GC content between 40% and 60%, no homo-polymer runs longer than two, no self-complementary stretches longer than 3, and be comprised of sequences not present in a human genome reference. Zipcodes can be of sufficient length and comprise sequences that can be sufficiently different to allow the identification of each nucleic acid (e.g., oligonucleic acids) or peptides based on zipcode(s) with which each nucleic acid or peptides is associated.

As used herein, the term "Y-adapter" generally refers to adapters with two DNA strands, part of which are not complementary to each other, thereby forming a fork of single-stranded DNA arms. The non-complementary arms of the Y-adapter can contain different elements such as identifiers, sequencing adapters, primer binding sites etc. On the top end of the Y-shape, one arm of the Y is different from the other arm of the Y. The bottom end of the Y-shape is double stranded (i.e. contains complementary strands). As used herein, Y-adapter and Y-shaped adapters are the same.

The attachment of the adapters to DNA fragments may be effected by ligating the Y-adapters to one or both 5'- or 3'-ends of the DNA fragments and then optionally carrying out an initial primer extension reaction, in which extension products complementary to the immobilized oligonucleotides are formed. This step may comprise an amplification step for multiplying the adapter-fragment-constructs. The forked or Y- adapters can be ligated to both ends of the DNA fragments by a DNA ligase. Only the double-stranded bottom end of the Y-adapter is able to ligate to the fragments DNA.

For use in the present disclosure, the Y-adapter may be ligated to both ends of the double stranded DNA fragments, wherein one strand of the adapter DNA is ligated to one 5'-end of the DNA fragment and the other strand thereof may be ligated to the respective 3' end of the DNA fragment, and this may happen on both sides of the DNA fragment. The sequence of the Y-adapter can be determined by considering various factors, including but not limited to, the type of DNA sequencing technology or system used for the DNA fragments library; and the primers used for PCR process after or during the construction of the DNA fragments library.

As used herein, the term "transposome" generally refers to a complex that comprises an integration enzyme such as an integrase or transposase, and a nucleic acid comprising an integration recognition site, such as a transposase recognition site. In some examples, the transposase can form a functional complex with a transposase recognition site that is capable of catalyzing a transposition reaction. The transposase may bind to the transposase recognition site and insert the transposase recognition site into a target nucleic acid in a process sometimes termed "tagmentation." In some examples, one strand of the transposase recognition site may be transferred into the target nucleic acid. In some examples, a transposome may comprise a dimeric transposase comprising two subunits, and two non-contiguous transposon sequences. In some examples, a transposome may comprise a dimeric transposase comprising two subunits, and a contiguous transposon sequence.

Transposases may include, but are not limited to Mu, TnlO, Tn5, hyperactive Tn5 See Goryshin and Reznikoff, J. Biol. Chem., 273:7367 (1998). Some examples can include the use of a hyperactive Tn5 transposase and a Tn5-type transposase recognition site. See Goryshin and Reznikoff, J. Biol. Chem., 273:7367 (1998). Some examples can include a MuA transposase and a Mu transposase recognition site comprising R1 and R2 end sequences. See, Mizuuchi, K., Cell, 35: 785, 1983; Savilahti, H, et al., EMBO J., 14: 4893, 1995. For example, a transposase recognition site that forms a complex with a hyperactive Tn5 transposase (e.g., EZ-Tn5^{™} Transposase, Epicentre Biotechnologies, Madison, Wisconsin) may comprise the following 19b transferred strand (mosaic end or "ME") and non-transferred strands: 5' AGATGTGTATAAGAGACAG 3', 5' CTGTCT CTTATACACATCT 3', respectively.

### Method

Another aspect of the present disclosure provides a method for synthesizing an array of polymers on a substrate. The array of polymers may comprise at least 100, 500, 1,000, 2,000, 3,000, 4,000, 5,000, 6,000, 7,000, 8,000, 9,000, 10,000, 20,000, 30,000, 40,000, 50,000, 60,000, 70,000, 80,000, 90,000, 100,000, 150,000, 200,000, 250,000, 300,000, 350,000, 400,000, 450,000, 500,000, 600,000, 700,000, 800,000, 900,000, 1,000,000, 10,000,000, 20,000,000, 30,000,000, 40,000,000, 50,000,000, 60,000,000, 70,000,000, 80,000,000, 90,000,000, 100,000,000, 200,000,000, 300,000,000, 400,000,000, 500,000,000, 600,000,000, 700,000,000, 800,000,000, 900,000,000, 1,000,000,000, 2,000,000,000, 3,000,000,000, 4,000,000,000, 5,000,000,000, or more unique polymeric molecules. First, a substrate which may fit for the purposes of polymer synthesis may be provided. The substrate may comprise a plurality of distinct locations. Each of the locations may comprise at least one site that is capable of attaching a subunit of the polymers onto the substrate. Each location may be adjacent to at least one, two, three, four, five, or six other locations. Each location may or may not have the same size, shape, or area. In some cases, a certain percentage of the locations has the same or a different size, shape, and/or area, for example, greater than or equal to 10%, 20%, 30%, 40%, 50%, 60%, 70%, 80%, 85%, 90%, 95%, or 99% of the locations may have the same size, shape and/or area.

Next, a set of masks may be provided. Each mask of the set may be used for defining a different subset of distinct locations on the substrate. Each mask may comprise a plurality of openings, which define a pattern of active regions and inactive regions on the substrate. During polymer synthesis, subunits can be added onto the locations within the active regions.

The openings may take various shapes, regular or irregular, such as square, rectangular, triangular, diamond, hexagonal, and circle. Each mask may have its own design of openings, which defines a distinct pattern of active and inactive regions on the substrate. The openings may or may not be aligned in a single direction. Each opening may cover an integer number of distinct locations on the substrate. For each mask, the openings may or may not be of the same shape. For each distinct location on the substrate, the set of masks collectively may define a unique string of synthetic steps or embedding (i.e., a sequence of subunits to be introduced onto the substrate) used to form the polymers in that location. Each mask may be used for at least one synthetic step for forming the polymers. In some cases, the set of masks are designed such that each pair of strings of synthetic steps (or embeddings) used to form the polymers at two adjacent locations differ from each other by a maximum number of synthetic steps, for example, by at most 500, 400, 300, 200, 100, 90, 80, 70, 60, 50, 45, 40, 35, 30, 25, 20, 18, 16, 14, 12, 10, 9, 8, 7, 6, 5, 4, 3, or 2 synthetic steps. In some cases, two strings of synthetic steps used to form polymers at two adjacent locations differ from each other by one and only one synthetic step. For example, each pair of embeddings used to synthesize neighboring polymers in two adjacent locations differs by one and only one exposure/non-exposure step.

For each mask, a certain percentage (e.g., 1%, 2%, 3%, 4%, 5%, 6%, 7%, 8%, 9%, 10%, 20%, 30%, 40%, 50%, 60%, 70%, 80%, 85%, 90%, 95% or more) or all of the openings may have the same length and/or width. In some cases, the length of the openings may be the same as the substrate. In some cases, the length of the openings may be less than that of the substrate such that one mask is only capable of masking a portion of the substrate. In cases where all of the openings have the same length, their widths may vary and one or more of the openings may or may not have the same width. For example, the width of the openings may be greater than or equal to about 1 nm, 10 nm, 50 nm, 100 nm, 250 nm, 500 nm, 750 nm, 1 µm, 2 µm, 3 µm, 4 µm, 5 µm, 6 µm, 7 µm, 8 µm, 9 µm, 10 µm, 20 µm, 40 µm, 60 µm, 80 µm, 100 µm, 200 µm, 300 µm, 400 µm, 500 µm, 600 µm, 700 µm, 800 µm, 900 µm, 1,000 µm, or more. In some cases, the width of the openings may be smaller than or equal to about 50 mm, 10 mm, 1,000 µm, 900 µm, 800 µm, 700 µm, 600 µm, 500 µm, 400 µm, 300 µm, 200 µm, 100 µm, 90 µm, 80 µm, 70 µm, 60 µm, 50 µm, 40 µm, 30 µm, 20 µm, 10 µm, 8 µm, 6 µm, 4 µm, 2 µm, 1 µm, or less. In some cases, the width of the openings may be between any of the two values described herein, for example, 12 µm.

The length of the openings may vary. In some cases, each of the openings has a length of greater than or equal to about 1 µm, 10 µm, 25 µm, 50 µm, 75 µm, 100 µm, 200 µm, 400 µm, 600 µm, 800 µm, 1,000 µm, 2,000 µm, 3,000 µm, 3,500 µm, 4,000 µm, 4,500 µm, 5,000 µm, 5,500 µm, 6,000 µm, 7,000 µm, 8,000 µm, 9,000 µm, 10,000 µm, or more. In some cases, the length of the opening may be smaller than or equal to about 50,000 µm, 25,000 µm, 10,000 µm, 8,000 µm, 7,000 µm, 6,500 µm, 6,000 µm, 5,500 µm, 5,000 µm, 4,500 µm, 4,000 µm, 3,000 µm, 2,000 µm, 1,000 µm, 800 µm, 600 µm, 400 µm, 200 µm, 100 µm or less. In some cases, the length of the openings may be between any of the two values described herein, for example, 4,900 µm.

To synthesize polymers having multiple segments, more than one set of masks may be provided and each set of masks may be used for synthesizing, for example, a specific segment of the polymers. For example, a first set of masks having openings of the same length but different widths may be used for forming a first segment of the polymers and a second set of masks having openings of the same width but different lengths may be used for forming a second segment of the polymers. The openings of the first set and the second set of masks may be aligned in a first direction and a second direction, respectively, and the first and the second directions can be orthogonal to each other. In some cases, the same set of masks for the first segment synthesis may be used to form the second segments of the polymers by rotating the masks 90 degrees. A third set of masks (or a separate mask) may be used in some situations for forming a third segment (e.g., a known sequence of polymers commonly shared by all the polymers) of the polymers, which mask(s) may be designed to subject all the locations to the polymer synthesis.

The mask can be formed of various materials, such as glass, silicon-based (e.g., silica nitrides, silica), polymeric, semiconductor, or metallic materials. In some cases, the mask comprises lithographic masks (or photomasks). Thickness of the mask may vary. In some cases, the mask may have a thickness of greater than or equal to 1 µm, 10 µm, 50 µm, 100 µm, 250 µm, 500 µm, 750 µm, 1 millimeter (mm), 2 mm, 3 mm, 4 mm, 5 mm, 6 mm, 7 mm, 8 mm, 9 mm, 10 mm, 15 mm, 20 mm, 25 mm, 30 mm, 35 mm, 40 mm, 45 mm, 50 mm, or more. In some cases, the mask may have a thickness of less than or equal to about 500 mm, 250 mm, 100 mm, 50 mm, 40 mm, 30 mm, 20 mm, 10 mm, 8 mm, 6 mm, 4 mm, 2 mm, 1 mm, 900 µm, 800 µm, 700 µm, 600 µm, 500 µm, 400 µm, 300 µm, 200 µm, 100 µm, or less. In some cases, thickness of the mask may be between any of the two values described herein, e.g., about 7.5 mm.

In one aspect, methods are provided to detect the distribution of a biomolecule in a two dimensional space. In some embodiments, the biomolecule may be made to react with the nucleic acid zipcodes. Zipcodes that have reacted with the biomolecule may then be sequenced or otherwise detected. Because the zipcodes encode their own locations, by detecting zipcodes, the biomolecule's spatial distribution can then be determined accordingly. Therefore, it is desirable to obtain high resolution zipcode arrays that can be decoded with high accuracy.

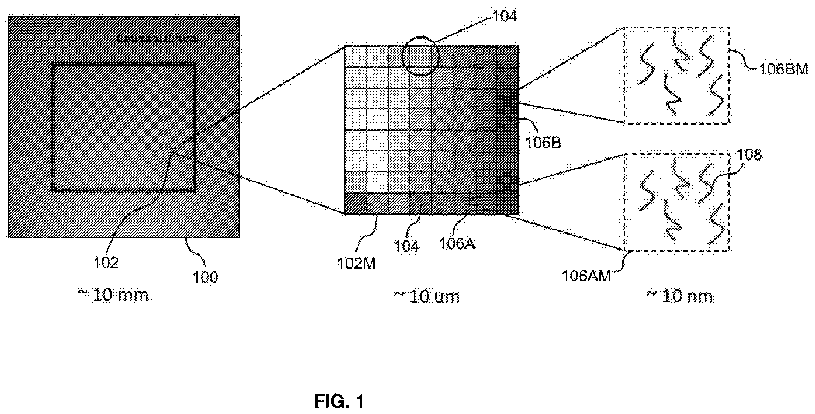

Turning now to FIG. 1, an example zipcode array chip 100 is shown. The zipcode array chip may be a square of about 10 mm in length. Zipcode array chip 100 may comprise a plurality of locations102. A location 102M may be a square of about 100 µm in length. The location 102M may comprise a plurality of sub-locations 104. Sub-locations 104 may be micron-scale features. Each zipcode array chip may comprise millions of micron-scale features. Each sub-location 104 may be a square of about 10 µm in length. Each sub-location 104 may comprise a plurality of mini-locations 106A and 106B, which may be a square of about 10 nm in length. A mini-location 106AM may comprise a plurality of identical, determinable sequences. The determinable sequence may comprise DNA, RNA, peptide, other polymer sequence or a mixture thereof. DNA, or polymer, will be used hereinafter as the determinable sequences to illustrate the principles of the present disclosure. As described *vide supra*, a zipcode generally refers to a type of biosequences, such as oligonucleotide molecules, that encodes its own positional information, spatial information, or locations.

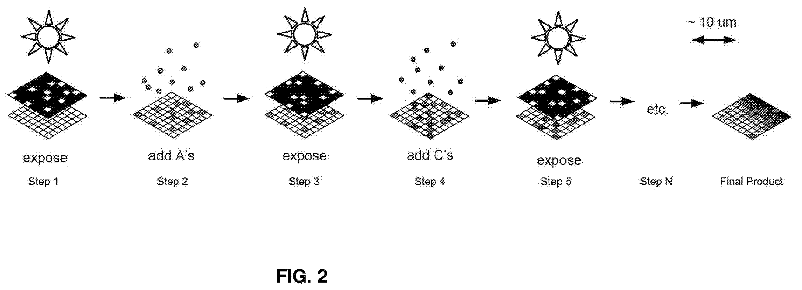

FIG. 2 illustrates the fabrication of a DNA zipcode array using photolithographic processes using masks (the top object in each step of FIG. 2) over a substrate (the bottom objection in each step of FIG. 2). Many process, such as spotting, inkjet printing, can be used to manufacture DNA zipcode arrays. As shown in FIG. 2, the openings (shown as white rectangular blocks) in the mask are used to synthesize the polymeric molecules, such as DNA's (i.e., when the mask is placed above the substrate, locations under the openings are to be exposed and subjected to polymer synthesis). Each opening has a selective size, for example, a minimum width of 5 µm or 1 µm or other sizes, and can cover one or more locations on the substrate, depending upon, e.g., the dimension and area of each individual location. The mask is designed such that when it is aligned with respect to the substrate, selected locations on the substrate can be activated and subunits can be added thereon.

Next, a computer executable logic may be provided and used to (i) select a mask to overlay the substrate; and (ii) select one or more subunits to be introduced onto each location on the substrate using the mask. The computer executable logic that selects the mask the one or more subunits is configures to generate the polymer array(s). Each polymer synthesized on (and thus immobilized at) a distinct location on the substrate may have a unique sequence (or a string of subunits). Each polymer immobilized at a distinct location may differ from another immobilized at adjacent distinct locations in the sequence by a maximum number of subunits, for example, by at most 500, 400, 300, 200, 100, 90, 80, 70, 60, 50, 45, 40, 35, 30, 25, 20, 18, 16, 14, 12, 10, 9, 8, 7, 6, 5, 4, 3, or 2, including substitutions, insertions, deletions, and/or translocations of single subunits. Subsequently, polymer synthesis may be performed using the selected masks and strings of subunits.

Various techniques can be used for synthesizing the polymers on the substrate, for example, chemical synthesis, electrochemical synthesis, or photoelectrochemical synthesis. In some cases, a light-directed synthesis is employed. A light source may be provided. The light source may be capable of performing the light-directed synthesis of the polymeric molecules on the substrate. The light source may provide various forms of radiations, such as visible light, ultraviolet light (UV), infrared (IR), extreme ultraviolet lithography (EUV), X-ray, electrons, and ions. The light source can provide a single wavelength, e.g. a laser, or a band of wavelengths. In some cases, the light beam provided by the light source may be in the range of ultraviolet to near ultraviolet wavelengths. A mask may be provided and positioned along an optical path between the light source and the substrate.

For example, in step 1 of FIG. 2, a first mask is used when the substrate is exposed to light. Then in step 2, exposed zipcodes on the substrates may be extended by one nucleic acid A. In step 3, a second mask is used when exposed to light so that exposed zipcodes on the substrates may be extended by one nucleic acid C in step 4. Then the ensuing step 5 uses a third mask to continue the synthesis. After multiple cycles, the final product is produced so that the zipcodes are form on the substrate and each zipcode can be determined later to reveal its location (in this case a two dimensional array).

As described above and elsewhere herein, multiple synthetic steps may be included in the whole polymer synthetic process, and in some cases, for each individual step, there is one and only one mask that is selected and placed along the optical path between the substrate and the light source. In some cases, to synthesize polymeric molecules with pre-defined sequences of subunits, a set of masks can be used and the combination of the masks determines a set of strings of synthetic steps (a series of exposure and non-exposure steps) for all of the locations on the substrate. An example multi-step synthetic route of polymer arrays is shown in steps of FIG. 2.

As provided herein, a computer system may be utilized to generate a mask design file for producing physical masks for use in the synthetic reactions. The computer system may comprise a computer readable medium, which may comprise codes that, upon execution by one or more computer processors, implements a method for generating the mask design file. In some cases, a mask set may be designed such that all pairs of strings of synthetic steps for forming polymers in adjacent zipcodes differ from each other by at most 500, 400, 300, 200, 100, 90, 80, 70, 60, 50, 45, 40, 35, 30, 25, 20, 18, 16, 14, 12, 10, 9, 8, 7, 6, 5, 4, 3, or 2 synthetic steps. For example, one zipcode may differ from any adjacent zipcodes by one and only one synthetic step.

During the manufacturing of the zipcode arrays, synthetic errors can occur so that the synthesized polymer or DNA sequences on the substrate are different from the desired sequences. In one aspect of the present disclosure, methods are provided to address expected errors during manufacturing and sequencing (determination of zipcodes) steps.

In some cases, misalignment of the masks relative to the substrate may occur and cause errors during synthesis, resulting in a mismatch between actual and desired polymeric sequences. In most instances, such misalignment causes errors where neighboring embeddings differ from each other at certain synthetic steps.

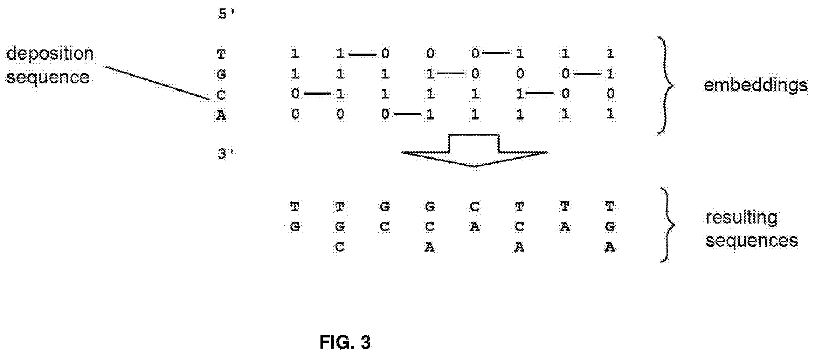

As used herein, the term "brown codes" generally refer to zipcode sequences that differ from their neighbors or adjacent zipcodes by exact one synthesis step (one bit), thereby can result in one base (or character) difference between the zipcode sequences. These codes are roughly the same length. FIG. 3 shows examples of few brown codes sequences. A brown code constraint as used herein generally refers to the zipcode selection criteria to select brown codes as the choice of zipcodes for adjacent embeddings in an array. This restraint may reduce the error rate of zipcodes due to synthesis errors.

To reduce the risk of errors caused by the misalignment, a set of embeddings may be generated to minimize the overall number of differences between neighboring embeddings. For example, the neighboring embeddings may differ from each other by exactly one change. An example set of embeddings and resulting polymeric sequences are shown in FIG. 3.

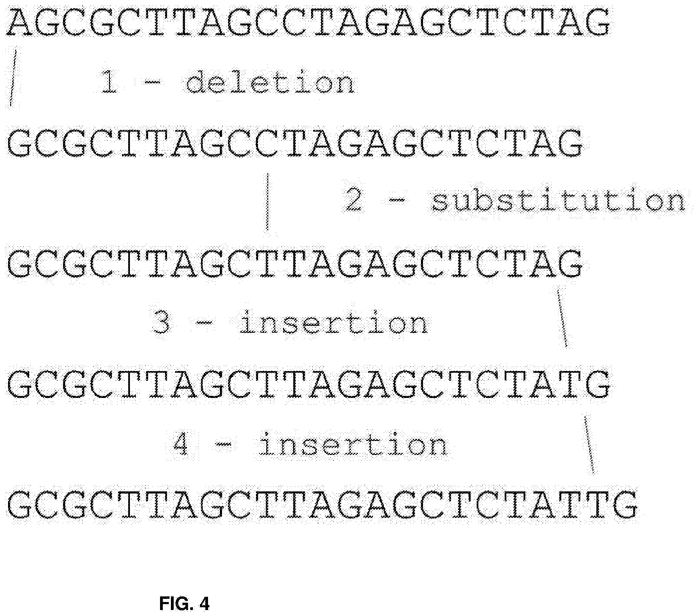

DNA zipcodes may be error correcting. This can be accomplished, for example, by ensuring that different zipcodes on the zipcode array have edit distances of at least 3, such as, for example, an edit distance of 4, as shown in FIG. 4. The edit distance between two sequences is the minimum number of changes (e.g., insertions, deletions, or substitutions) needed to convert one sequence into another sequence. For example, FIG. 4 shows an example of error correcting DNA zipcodes (AGCGCTTAGCCTAGAGCTCTAG and GCGCTTAGCTTAGAGCTCTATTG) which have an edit distance of 4.

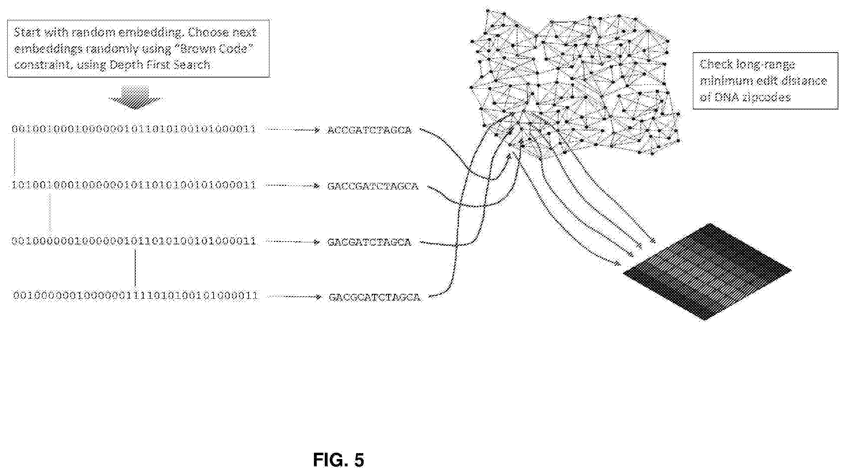

In some cases, it may be desired to have a plurality of polymers synthesized, which polymers have (1) roughly equal lengths, and (2) higher long-range minimum edit distance, The long-range minimum edit distance, as used herein, dictates for some given D, if two polymers are ≥D locations apart on the substrate, their edit distance must be ≥D. The synthesized polymers can be short or long. In cases where short sequences are needed, the synthetic route may comprise (i) generating embeddings of all possible lengths that meet the abovementioned two constraints, i.e., all resulting polymers have substantially the same length and higher long-range minimum edit distance, and (ii) using the shortest length that yields enough polymers for synthesis. An example method is illustrated in FIG. 5, which method starts with initializing an empty list of embedding and selecting a random embedding of length n (a string of 0's and 1's). Then, a candidate embedding is randomly chosen using the first constraint. After the number of embeddings included in the list reached a predetermined value, the second constraint is applied and the embeddings failing to meet this constraint are removed from the list. Such generated embeddings can then be used for synthesizing polymer having a single segment. FIG. 5 shows a design of a zipcode array.

In some embodiments, starting with random embedding, first choose next embeddings randomly using "brown code" constraint and using Depth First Search. Then the zipcodes may be checked for long-range minimum edit distance of DNA zipcodes.

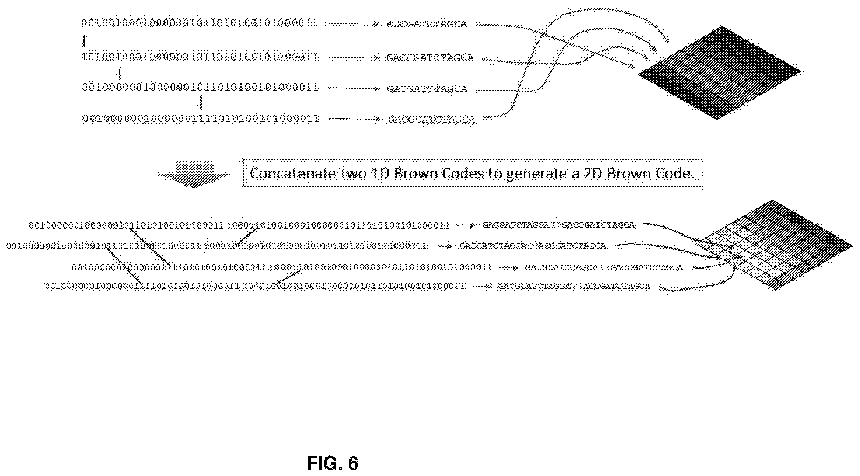

In some cases, two or more (e.g., 2, 3, 4, 5, 6, 7, 9, or 10) of the generated embeddings are concatenated to form a new set of embeddings that can be used for synthesizing polymers having multiple segments. Additionally or alternatively, a common known embedding with a much shorter length than (e.g., a string of 0's and 1's of length less than 2, 3, 4, 5, 6, 7, 8, 9, or 10) and distinguished from the concatenated embeddings may be inserted into neighboring concatenated embeddings to separate them, and each of the concatenated embeddings may correspond to a segment of the polymers. For example, as shown in FIG. 6, each embedding is generated by concatenating two previously generated embeddings (FIG. 5) and inserting a common string (i.e., TT or CCC or GGG) between the concatenated embeddings. Each of the newly formed embeddings comprises three sections each corresponding to a single segment of the resulting polymers, e.g., an upper segment, a middle segment and a lower segment. The upper and the lower segments may encode the x- and y-coordinate respectively, and the middle segment is used for separating the upper and the lower segments. In some cases, the sets of embeddings prior to and after the concatenation are called 1D and 2D embeddings and the generated 1D and 2D embeddings can be separately used to design masks for synthesizing polymers that have a single and multiple segments respectively. FIG. 5 illustrates a design process for a zipcode array (implemented at Centrillion Technologies, Inc. for its Arcadia and Yosemite zipcode arrays).

The upper or lower segments of zipcodes may be 4-24 bases in length, 8-20 bases in length, 12-16 baes in length, or no more than 16 bases in length. If a single zipcode may be used for determine the spatial information of biomolecule as well.

Optionally, the encoding of two dimensional zipcodes can be done by two sequence segments: one for the x coordinate and the second for the y coordinate. In some cases, a separator such as the sequence "GGG" may be inserted between the x and y sequences to aid decoding. In some cases, two 1D brown codes can be concatenated by a common string (e.g., FF or CCC or GGG) to generated a 2D brown code to encode x or y-coordinate, as shown in FIG. 6.

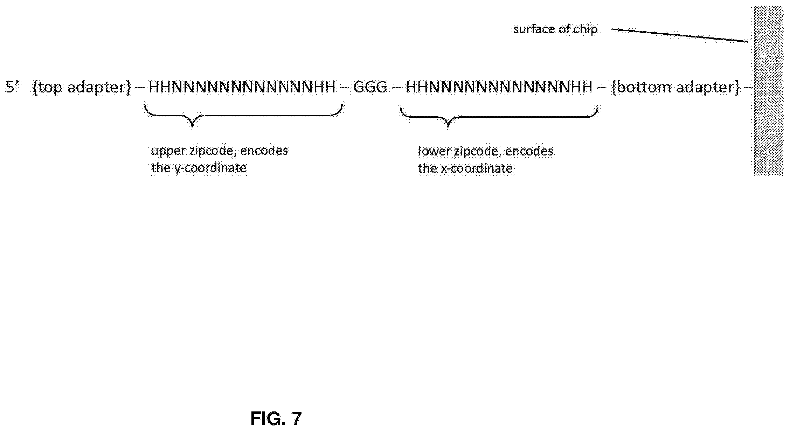

FIG. 7 shows the design of the "Yosemite" zipcode array currently manufactured at Centrillion Technologies, Inc. (Palo Alto, CA). This zipcode array has sequences with long-range minimum edit distance of 5. In this example, the lower zipcode and upper zipcode both have max lengths of 16 bases. Other lengths are possible. In this example, the top adapter is at the 5' end of each zipcode sequence; the bottom adapter is at the 3' end of each zipcode sequence and is attached to the surface of a chip; a sequence of GGG separates the upper zipcode and the lower zipcode; the upper zipcode encodes the y-coordinate of the zipcode sequence; the lower zipcode encodes the x-coordinate of the zipcode sequence, the x- and y-coordinates determines the spatial location of the zipcode sequence on the zipcode array. As used herein, the term "coordinate" generally refers to numerical values or symbolic representations of a specific position on a 2-dimensional surface or in a 3-dimensional body. For example, a 2-dimensional surface can be defined according to X and Y coordinates according to a coordinate system, wherein the X and Y coordinates are the horizontal and vertical addresses of any position or addressable point, respectively. As used herein, the term "coordinates of contact" generally refer to the coordinates of a specific position on a 2-dimensional surface or in a 3-dimensional body, at which position the zipcode array makes contact with another entity, including for example, a sample, a target molecule, or a molecule to be analyzed.

The same 36 masks were used for synthesizing both the lower and upper zipcodes (rotating 90 degrees for upper zipcodes). The chip has 5,000 different sub-zipcodes (x and y), which yields 25 million 2 µm zipcodes on a 10mm × 10mm chip. The zipcodes can be linked to top and bottom adaptor sequences. These adaptor sequences are added to facilitate biochemical reactions on surface. For example, in one format of the Yosemite chip (available at Technologies, Inc., Palo Alto, CA), the bottom adaptor is a sequencing adaptor for preparing a sequencing library and the top adaptor is a primer for cDNA synthesis to catch RNA molecules. The probes can be in 5' to 3' orientation or 3' to 5' orientation. Synthesis can be in 5' to 3' orientation or 3' to 5' orientation. In some cases, the probes are synthesized in 3' to 5' orientation and they are then flipped to result in 5' to 3' (from surface) orientation.

Zipcodes, once sequenced, can be decoded for its positional information (x and y location) using a software that comparing the designed zipcodes and putative zipcodes identified. Because sequencing and synthesis errors can occur, the decoding software may use approximate string search to determine the zipcode match and the resulting positional information. Centrillion's PostMark^{™} zipcode decoding software is available from Technologies, Inc. (Palo Alto, CA) to decode the Yosemite zipcode array.

In one embodiment, extra zipcodes may be designed but not used in the actual chip synthesis. During decoding, these designed but not used codes are also compared with putative zipcodes from sequencing reactions. A match of the unused zipcodes can indicate a zipcode decoding error. Therefore, these extra zipcodes can be used to assess the stringency of the decoding algorithm.

The following examples illustrate the application of a Yosemite zipcode array.

### Example 1: Preparing a Zipcode Array

In this example, the Yosemite chip described above was prepared.
*Generating Spatial Oligo Array Hydrogel*

Printing oligonucleotide, can be, for example:
/5Acryd/TT TTU UUU U/iSpC3//iSp18/ GAC TCG TAA TAC GAC TCA CTA TAG GGA CAC GAC GCT CTT CCG ATC T

The printing oligo (IDT) consists of a 5' acrydite group that attaches the oligo to the hydrogel, a Uracil-Specific Excision Reagent (USER) enzyme site, a C3 spacer to reduce unwanted background reverse transcription noise, a T7 promoter, and a sequencing adapter sequence. Printing uses an oligo array chip ("Yosemite" Zipcode array, Centrillion Technologies, Inc., Palo Alto, CA), as template and extends the oligo to contain a spatial zipcode, for example, a 26 mer, a 27 mer, a 28 mer, a 29 mer, a 30 mer, etc., after the sequencing adapter sequence on the printing oligo, and followed by a poly(T) tail designed to capture mRNA in the tissue specimen.

The extended oligonucleotide on the printed hydrogel, can be, for example:
/5Acryd/TT TTU UUU U/iSpC3//iSp18/ GAC TCG TAA TAC GAC TCA CTA TAG GGA CAC GAC GCT CTT CCG ATC T [30 mer_zipcode] GAT TTT TTT TTT TTT

### TTT TTT TVN

A 6% acrylamide gel containing 50 µM of printing oligo is casted on a silanized glass slide. 10 µL of the printing oligo mixture is covered with a 15-mm diameter circle cover slip and let polymerize at 25 °C for 30 mins. The cover slip is carefully lifted and excess unpolymerized oligo is rinsed away with MilliQ water. A 1cm × 1cm oligo array chip is stuck on a 1 in × 1 in acrylic base with double sided tape. Two 15mm × 15mm frame seal (Bio Rad) are stuck on top of each other and onto the acrylic base to surround the oligo array chip. 100 µL of printing solution with 1× Thermopol Buffer (NEB), 0.2 µg/µL BSA, 200 µM dNTP mixture, and 0.32U/µL Bst DNA polymerase, large fragment (NEB) was added on top of the oligo array chip. The glass slide with the oligo hydrogel is stacked on top of the chip with the gel side facing the chip. A 1 in × 1 in PDMS cushion is added on top of the glass slide and the whole cassette is held together by a 2" binder clip. The whole setup is incubated in a humidified container at 55 °C for 3 hrs. The two surfaces are separated by removing the binder clip, PDMS cushion and immersing the oligo hydrogel and oligo array chip, still stuck together, in 95 °C MilliQ water for 15 mins. The oligo hydrogel is released from the chip by carefully lifting the glass slide away from the chip.

### Example 2: Detecting Spatial Distribution of RNA molecules in Tissue Sections

In this example, the Yosemite chip described in Example 1 above is used to detect the spatial distribution of RNA molecules.

### Generating Spatial Oligo Array Hydrogel

A procedure that is the same as or similar to the one disclosed in Example 1 is used in Example 2.

### Capturing mRNA from Tissue Section with Printed Oligo Hydrogel

Purchased fresh frozen sections of mouse olfactory bulb or mouse embryo E13 was thawed at room temperature for 5 mins and fixed for 10 mins in 4% formaldehyde (diluted 1:9 from 36.5-38.0 stock solution (Sigma-Aldrich) in 1× phosphate buffered saline (PBS)). After fixation, the sections were rinsed with 1× PBS and 500 µL of pre-warmed 0.1% pepsin (Sigma-Aldrich) dissolved in 0.1M HCl was added on top of the tissue section. The section was incubated in a humidified chamber at 37 °C for 6-10 mins (for olfactory bulb: 6 mins; for embryo: 10 mins). The solution was topped off and rinsed with 1× PBS. Excess liquid was dabbed dry with Kimwipe^{®}. 20 µL of reverse transcription solution was added onto the printed oligo hydrogel and the permeabilized section was stacked on top of the gel carefully avoiding air bubbles. The reverse transcription solution contained 1× First Strand buffer (Invitrogen), 5 mM dithiothreitol (DTT) (Invitrogen), 500 µM dNTP mix, 50 ng/µL Actinomycin D (Sigma-Aldrich), 1% DMSO (NEB), 20U/µL Superscript III (Invitrogen) and 2U/µL RNaseOUT (Invitrogen). The section and oligo hydrogel was incubated in a humidified chamber at 42 °C overnight (15-16 hrs).

### cDNA Library Preparation and Sequencing

The tissue section and oligo hydrogel is removed from the humidified chamber and immersed in 0.1× SSC buffer for 2 mins. The sections were lifted from the oligo hydrogel slide and excess liquid was dabbed dry with Kimwipe^{®}. The oligo hydrogel is then scraped off from the glass slide and into a 0.2 mL polymerase chain reaction (PCR) tube. 20 µL of oligo release solution containing 1.1x Second Strand Buffer (Invitrogen), 250 µM dNTP mix, 0.1U/µL USER Enzyme (NEB) was added and incubated at 37 °C for 2 hrs. 5 µL of second strand solution containing 3× First Stand Buffer, 3.7U/µL DNA polymerase I (NEB), 0.18U/µL RNaseH (NEB), 20U/µL T4 DNA ligase, and 0.5 mM ATP was further added to the hydrogel and incubated at 16 °C for 2 hrs. 2 µL of T4 DNA polymerase was additionally added and the reaction was incubated at 16 °C for another 20 mins. The reaction was stopped by adding 25 mM EDTA and the supernatant was transferred to a new tube. The sample was purified using Agencourt AMPure XP beads (Beckman Coulter) with beads to sample ratio of 0.75: 1 and eluted in nuclease-free water (IDT). The sample was mixed with In Vitro Transcription solution containing 1× T7 Reaction Buffer (Ambion), 7.5 mM of each NTP (Ambion), 1× T7 Enzyme Mix (Ambion) and 1U/µL SUPERaseIN (Ambion). The sample was incubated at 37 °C for 15-16 hrs.

The sample was purified using Agencourt AMPure XP beads with beads to sample ratio of 0.75: 1 and eluted in nuclease-free water (IDT). 0.5 µM of sequencing ligation adapter (IDT) was added to the sample and heated at 70 °C for 2 mins and immediately placed on ice. Adapter ligation solution containing 1× T4 RNA Ligase Reaction Buffer (NEB), 20U/µL T4 RNA Ligase2, truncated (NEB), 4U/µL RNase Inhibitor, Murine (NEB) is added to the sample and incubated at 25 °C for 1 hr. The sample was purified using Agencourt AMPure XP beads with beads to sample ratio of 0.75: 1 and eluted in nuclease-free water (IDT). 1 µM of RT primer (IDT) and 0.5 mM dNTP mixture was added to the sample and heated at 65 °C for 5 mins and then immediately placed on ice. Reverse transcription solution containing 1× First Strand Buffer, 5 mM DTT and 10U/µL Superscript III and 2U RNaseOUT was added to the sample and the reaction was incubated at 50 °C for 1 hr. The sample was purified using Agencourt AMPure XP beads with beads to sample ratio of 0.75: 1 and eluted in nuclease-free water (IDT).

To determine how many PCR cycles is needed to amplify the sample, 1/5 volume of the sample is added to a qPCR mixture containing 1× KAPA HiFi Reaction Buffer, 0.3 mM dNTP mix, 0.5 µM sequencing adapter 1, 0.5 µM sequencing adapter 2, 1× EVA Green (Biotium) and 0.5U/reaction KAPA HiFi DNA Polymerase. After the number of cycles is determined for the sample, the remaining sample is amplified with the same conditions as the qPCR reaction. The amplified sample or sequencing library was purified using Agencourt AMPure XP beads with beads to sample ratio of 0.75: 1 and eluted in nuclease-free water (IDT). The concentration of the library was quantified using KAPA Library Quantification Kits (KAPA Biosystems) per the manufacturer's protocol. Libraries were diluted to 2 nM and sequenced on the Illumina MiSeq platform using paired-end sequencing per the manufacturer's protocol.

### Sequencing ligation adapter:

/5rApp/AGA TCG GAA GAG CAC ACG TCT GAA CTC CAG TCA C/3ddC/
RT primer:
   GTG ACT GGA GTT CAG ACG TGT GCT CTT CCG ATC T
Sequencing adapter 1: 
AAT GAT ACG GCG ACC ACC GAG ATC TAC ACT CTT TCC CTA CAC GAC
GCT CTT CCG ATC T
Sequencing adapter 2: 
CAA GCA GAA GAC GGC ATA CGA GAT GTG ACT GGA GTT CAG ACG
TGT GCT CTT CCG ATC T
*Sequencing Alignment and Zipcode Decoding*

The read containing the gene information was first aligned to the mouse genome using Spliced Transcripts Alignment to a Reference (STAR) software. STAR is free open source software distributed under GPLv3 license and can be downloaded from the web address of <http://code.google.com/p/rna-star/>. The reads that were aligned were extracted and the corresponding read that contains the zipcode was decoded for its positional x, y coordinate information. The number of reads aligned to each gene was counted with htseq-count. The reads that aligned to a coding transcript were extracted and whenever a read that contains a coding transcript and the corresponding read contains a decoded zipcode, the information is written to a new file that contains the combined gene expression and positional information.

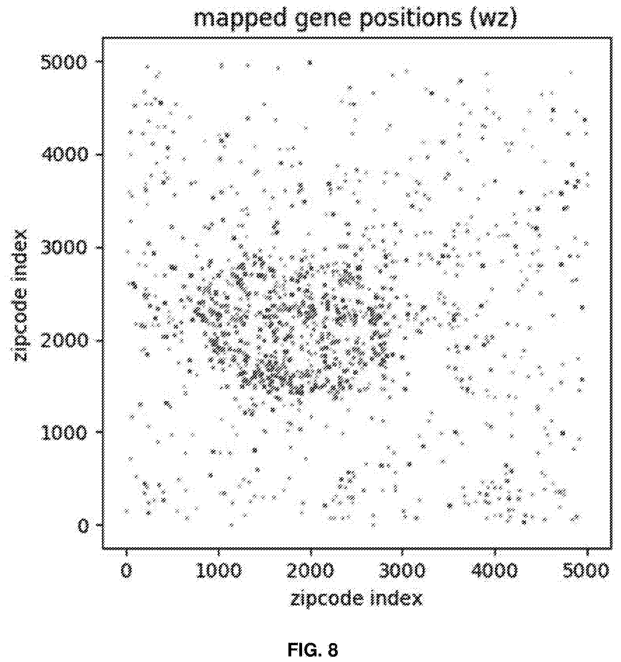

Results: FIG. 8 shows the 2-dimensional distribution of RNA molecules within and around a mouse brain section. The high density area matches with the position of the brain tissue.

### Detecting Spatial Distribution of biomolecules in Tissue Sections using Binding Partners

In another aspect, Zipcode Arrays are used to detect the two dimensional distribution of any molecules that can be detected by a binding partner such as an antibody, an aptamer, or a synthetic antibody mimics (SyAMs) (P. J. McEnaney, et al., "Chemically Synthesized Molecules with the Targeting and Effector Functions of Antibodies." Journal of the American Chemical Society, 2014 Dec 31; 136(52):18034-43. DOI: 10.1021/ja509513c, which is incorporated herein by reference).

The binding partner can be labeled with an oligonucleotide barcode or oligonucleotide tag. Antibodies can be readily conjugated with oligonucleotide barcode sequences (see, for example, "Antibody-Oligonucleotide Conjugate Preparation and applications," [online]. Retrieved from <http://www.solulink.com/products/white-papers/antibody-oligo-conjugate-preparation.pdf>, which is incorporated herein by reference). The oligonucleotide tag can be selected based upon its hybridization specificity to selected targeted sequence and its uniqueness (each tag may represent one antibody or one binding partner type/identity). The binding partners can be mixed with and react with, for example, a tissue section or transfer thereof. The stained tissue section can be then placed on top of a zipcode array or a copy of the zipcode array. The zipcodes can be made to react with the binding tags. For example, the tag oligonucleotides can contain a common sequence similar to the poly-A tail of the mRNAs. By binding with the common sequence of oligonucleotide tags, the zipcode can serve as a primer for an extension reaction to copy the tag sequences. In some cases, after the extension reaction to copy the tag sequences, some zipcode sequences can be linked with the binding partner (antibody) tag sequences. There are many methods to link tag sequences with the zipcode, some of which can be found elsewhere in this disclosure. In some cases, the tag oligonucleotides and the zipcodes can be ligated directly or through an intermediate. In some cases, the tag oligonucleotides can be used as templates in an extension reaction such that copies of the tag oligonucleotides can be added to the sequences to be analyzed.

Afterwards, the zipcodes can be sequenced to analyze the distribution of the binding partners' tags for their spatial distribution. This can be performed after an amplification reaction. The zipcode may provide the location of the binding partners and the tag nucleotide sequences may provide the identification of the binding partners. Many binding partners can be used at the same time to detect many different molecules at the same time. The method can be used to detect protein molecules, lipids, antigens, natural products, metabolites, and other biological molecules.

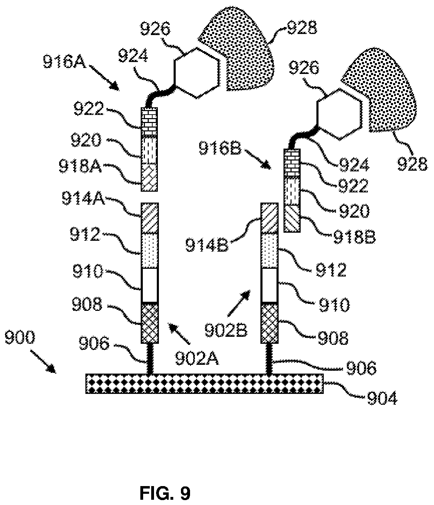

Turning now to FIG. 9, there is a zipcode array 900. Zipcode array 900 comprises a plurality of zipcodes 902A and 902B. Both zipcodes 902A and 902B are attached to the substrate 904 via linker 906. Both zipcodes 902A and 902B comprise a bottom adaptor 908, a coordinate zipcode 910 which encodes the coordinates of the specific location of the zipcodes 902A or 902B, respectively, and an upper adaptor 912. Zipcode 902A comprises a binding sequence 914A while zipcode 902B comprises a binding sequence 914B. As shown in FIG. 9, zipcode 902A binds to or recognizes binding partner 916A while zipcode 902B binds to or recognizes binding partner 916B. Both binding partner 916A and binding partner 916B comprise a detector 926 linked via linker 924 to an adaptor 922 and a barcode 920 that encodes for detector 926. Binding partner 916A comprises a binding sequence 918A that is recognizable by binding sequence 914A while binding partner 916B comprises a binding sequence 918B that is recognized by binding sequence 914B. As used herein, the term "recognizable" generally refers to specific interactions between two binding sequences, either between themselves or via a third party, such that the two binding sequences can have affinity to each other. For example, the binding pair of 914A and 918A may be ligated by a ligase because each has a sequence at least partially recognizable by the ligase for the ligation, or by a gap filling reaction due to their special proximity. The binding pair of 914B and 916B may hybridize together to form a complex due to the complementarity between their sequences. Detector 926 can detect and bind biomolecule 928 that may exist in a biological sample.

Therefore, when biomolecule 928 binds detector 926 to form the first complex, the first complex may be placed on the zipcode array 900. Nonbinding detectors 926 may be washed away. Due to close vicinity for binding sequence pairs 914A and 918A, and 914B and 918B, the binding partner 916A may bind to zipcode 902A and the binding partner 916B may bind to zipcode 902B. Then a sequencing reaction may be employed to produce a reporting sequence comprising both coordinate zipcode 910 and barcode 920. This reporting sequence then may report the spatial distribution of biomolecule 928 in the biological sample.

By analyzing the frequency of tags or in specific locations, it is possible to plot the distribution of interested or target molecules.

### Example 3: Spatial Analysis of Tumor Tissues

In this example, the zipcode arrays are used to spatially analyze tumor tissues.

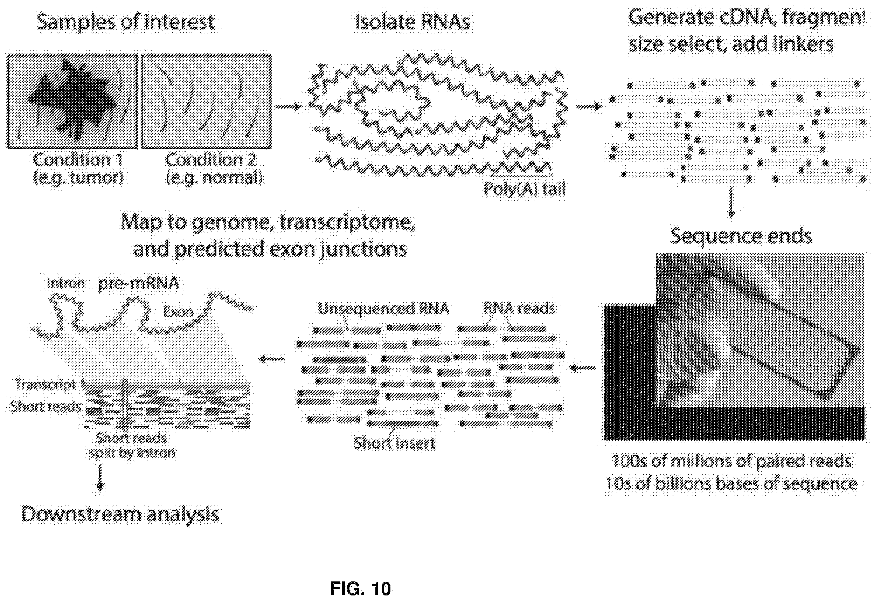

FIG. 10 shows an example process to analyze RNA sequences from samples of interest, e.g., comparison of tumor tissues and normal tissues. In some cases, RNAs with poly-A tails are isolated from samples of interest. The isolated RNAs are reverse-transcribed in to complementary DNAs (cDNAs), which are fragmented, size-selected, end modified (e.g., adding linkers to one or both ends of the fragmented cDNA). The fragmented and modified cDNA are sequenced by sequencing methodologies, e.g., generating paired reads to be analyzed. These reads can be further analyzed using know transcript from the original RNAs, knowledge about introns, pre-mRNA, etc., and mapped to genome, transcriptome, and predicted exon junctions, etc.

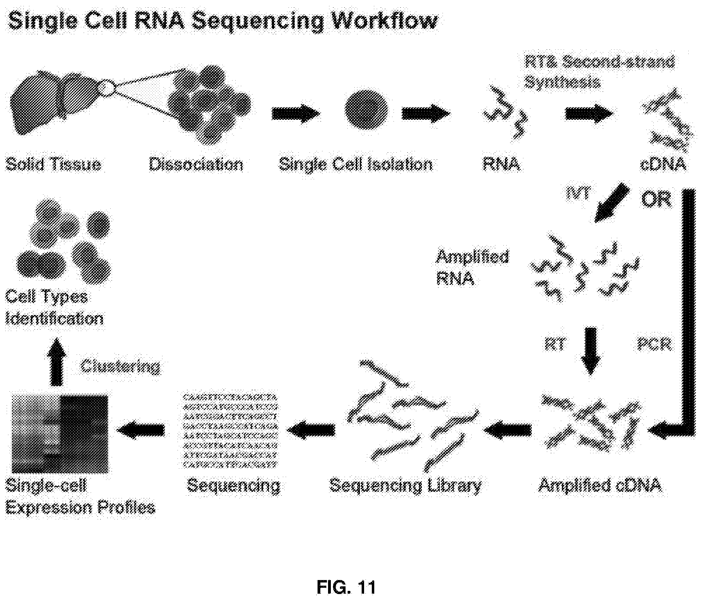

FIG. 11 shows an example process to analyze RNA sequences from a single cell. A solid tissue, e.g., liver tissue, is treated to dissociate the cells. Cells are separated and isolated. RNAs from a single cell are collected, and used as the templates for reverse transcription followed by second strand synthesis to produce double-stranded cDNA samples. Then amplified cDNA samples are made either using polymerase chain reaction (PCR) method or a combination of in vitro transcription (IVT) and reverse transcription (RT) methods. The amplified cDNA library is then sequenced. The sequencing results are analyzed to produce single-cell expression profiles. Further analysis produces cell types identifications when different expression profiles from different single cells are combined and analyzed.

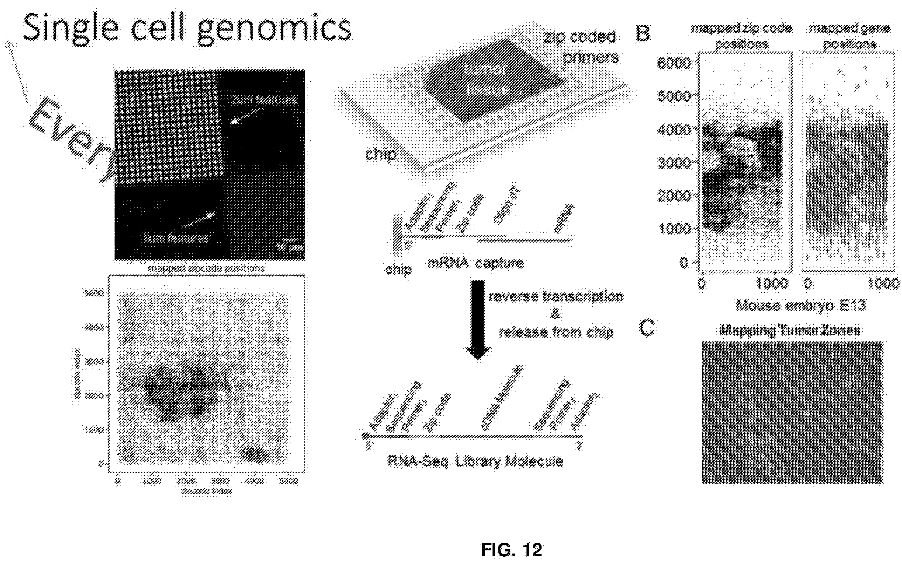

FIG. 12 shows an example process to analyze single-cell genomics. A zipcode code chip comprising zipcoded primers is provided. Each zipcode on the chip comprises, sequentially from the surface of the chip, a 5'-end adaptor, a sequencing primer, a zipcode coding for the spatial position of the zipcode on the chip, and an oligo dT tail on the 3'-end. A tissue sample, e.g., a tumor tissue, is placed on the zipcode. RNAs from the tissue sample comprises poly-A tails can be captured by the zipcodes on the chip, reverse transcribed, end modified, amplified to produce a RNA sequencing library comprising complementary DNA (cDNA) sequences from the corresponding RNAs from the tissue. Each cDNA sequence has a zipcode or a complementary of the zipcode attached. After sequencing the cDNA sequences, various results are obtained, including but not limited to: mapped zipcode positions for RNA sequences on the chip, mapped gene positions on the chip (hence, 2-dimensional distribution in the original tissue sample), mapped tumor zones (based on known sequences associated with tumor).

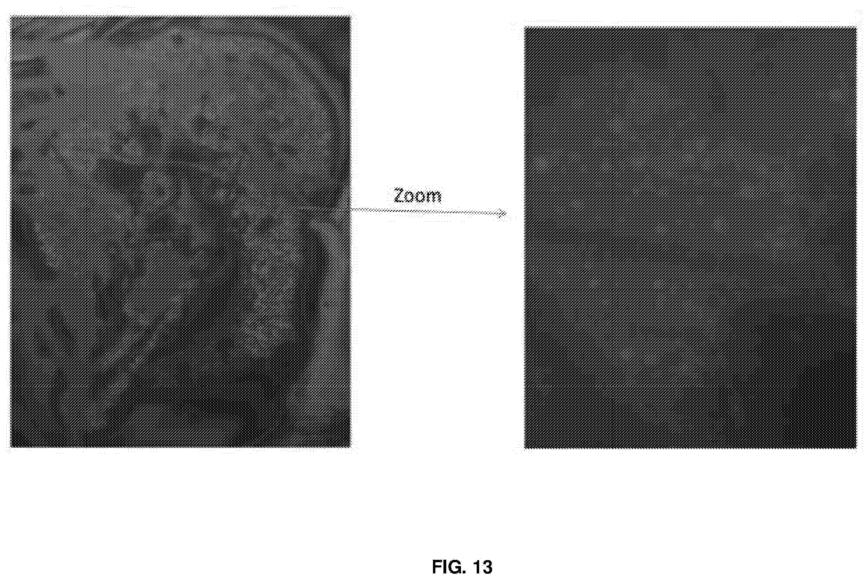

FIG. 13 shows an example spatial RNA sequencing analysis results, presenting a 2-dimensional distribution of RNA sequences showing, for example, relative amounts of the same sequence, or distribution of different sequences, etc. A magnified view of an area of interest is shown on the right panel, relative to the original mapping on the left panel.

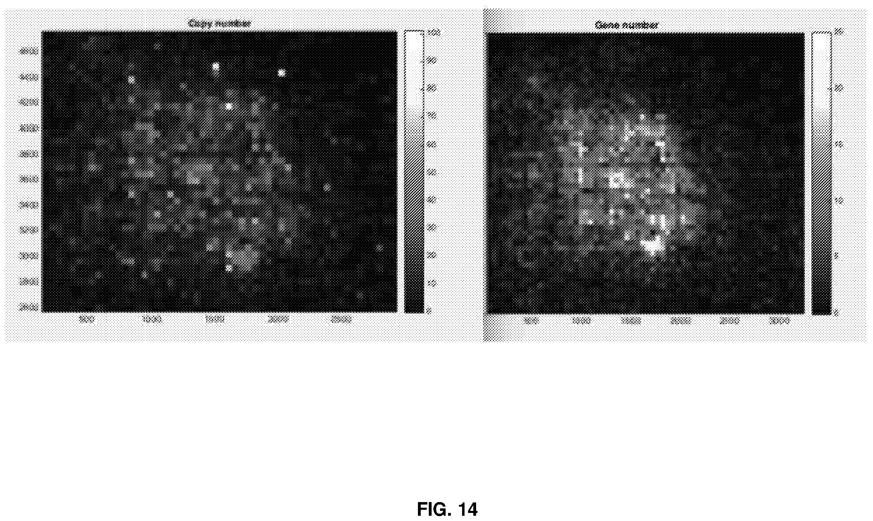

FIG. 14 shows other examples of spatial RNA sequencing analysis. The left panel shows distribution of different copy numbers of the same RNA sequence. The right panel shows distribution of gene numbers.

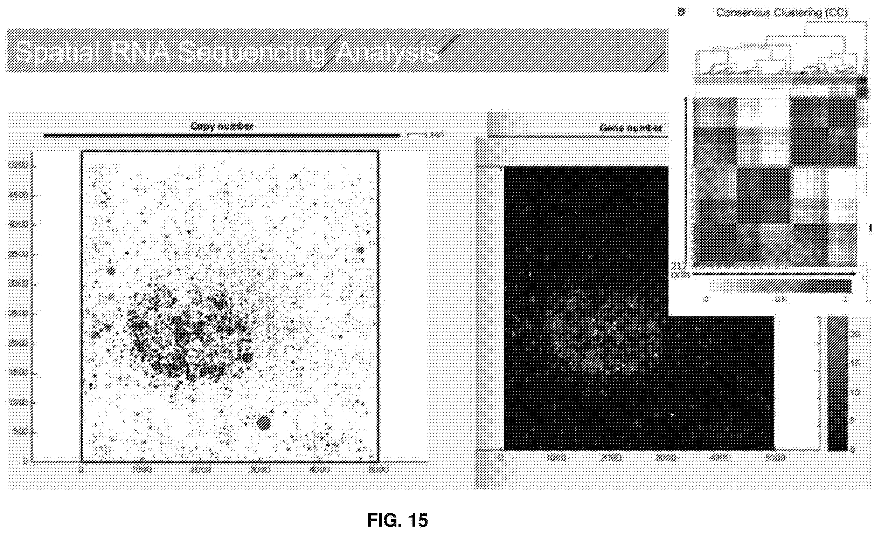

FIG. 15 shows still other examples of spatial RNA sequencing analysis. Panel B shows consensus clustering (CC) analysis of the distributions of copy numbers and gene numbers of a tissue sample.

Nucleic acids other than RNA can be analyzed spatially as well. In some examples, the method, kit and system of the present disclosure can be used to provide spatial profiling of the genome and epigenome in tissues, e.g., tumor tissues. In other examples, the method, kit and system of the present disclosure can be used to provide megabase sequencing analysis of very long nucleic acid sequences by scaffolding short reads obtained from the very long nucleic acid sequences and /or relying on long range sequencing contiguity.

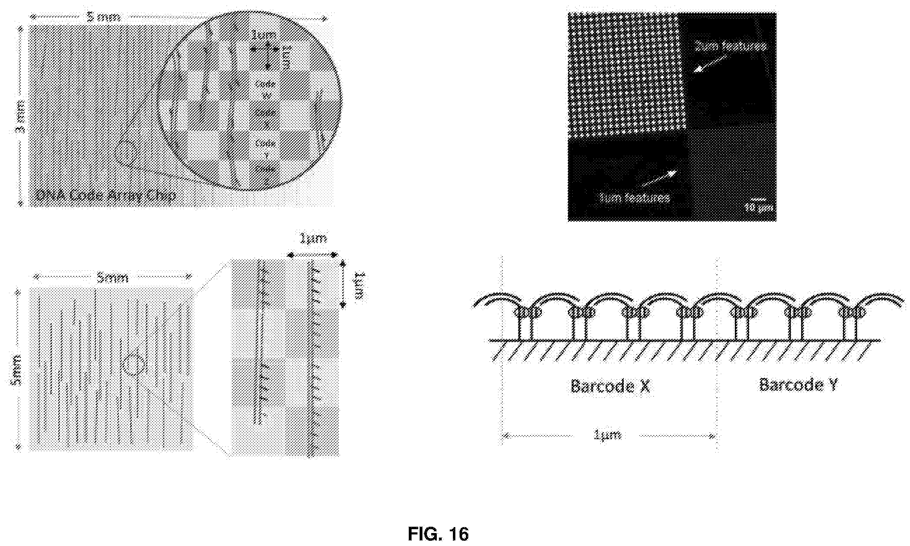

FIG. 16 shows an example of megabase sequencing. In this example, a zipcode array chip may be provided in the top left panel. Long nucleic acids may be stretched and placed on top of the zipcode array chip. The zipcode array chip (5 mm × 3 mm in size) may distinguish physical locations up to 1 µm × 1 µm dimensions, i.e., all zipcodes within the 1 µm × 1 µm dimension are the same, but are different from neighboring 1 µm × 1 µm dimensions. The lower left panel shows another configuration of the zipcode array chip, which is 5 mm × 5 mm in size, comprising 1 µm × 1 µm distinctive positions encoded by zipcodes. The top right panel shows a picture of a zipcode array chip having 1 µm × 1 µm distinctive positions (or features) and another zipcode array chip having 2 µm × 2 µm distinctive positions (or features). The bottom panel shows an example of dissection of a zipcode array chip with barcodes X within one distinctive position and barcodes Y within another distinctive position.

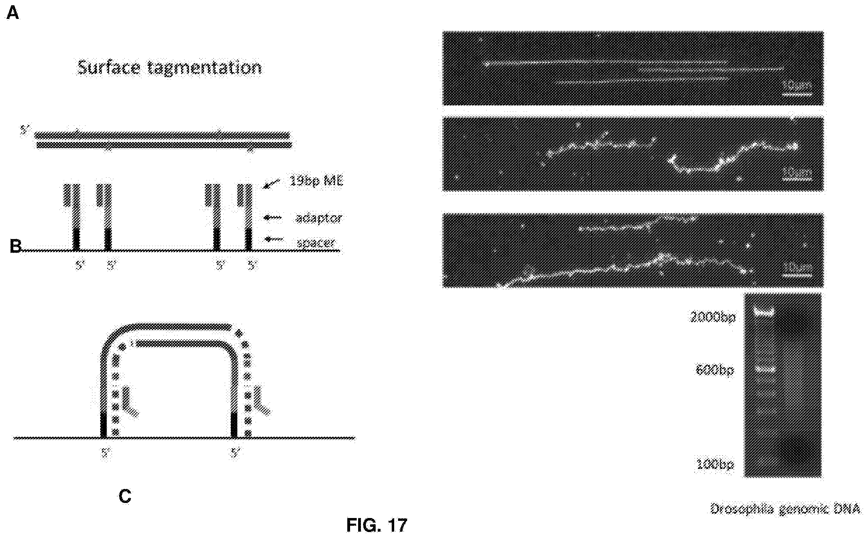

FIG. 17 shows an example of how to generate a sequencing library. A double stranded DNA molecule (Panel A) can be tagmented using immobilized oligonucleotides comprising a 19 bp mosaic end (ME) recognition sequence, an adaptor sequence (e.g., for priming purposes or provide a binding site for a primer) and a spacer at the 5' end that is covalently attached to the substrate surface. See Panel A of FIG. 17.

For example, a hyperactive Tn5 transposase (not shown) may be bound to immobilized oligonucleotides on the substrate surface. The oligonucleotide may contain 19 bp of the "mosaic end-recognition sequence" (shown as "ME" in FIG. 17) as well as the flanked adaptor for deep sequencing, or zipcode, to form a transposase complex. The mosaic end-recognition sequence, which is a tandem conjugate sequence of outside-end and inside-end recognition sequences in the insertion sequence of the wild-type Tn5 transposon, may be used for random integration into the target DNA on the substrate surface. If genomic or cDNA is incubated with this transposase complex, the fragmentation and the attachment of the target DNA to the adaptors may occur simultaneously. When the fragmentation and the tag-addition are finished, a gap in the non-transferred strand may be present due to the action of the transposase. This gap can be filled.

Further steps, such as, for example, denaturing, polymerase-catalyzed elongation, etc., may generate DNA fragments with adapters and zipcodes. See Panel B of FIG. 17.

Panel C of FIG. 17 shows various pictures taken. The first picture from the top shows stretched DNA on top of a zipcode array. The second and third pictures from the top show tagmented DNA on top of a zipcode array. The bottom picture shows the distribution of tagmented *Drosophila* genomic DNA fragments display after an agarose gel electrophoresis.

### Capturing mRNA from Tissue Section with Oligo Hydrogel

Oligo gel oligonucleotide:
/5Acryd/UUUUU/iSp18/AGACGTGTGCTCTTCCGATCTWSNNWSNNVTTTTTT TTTTTTTTTTTTTTVN

The oligo (IDT) consists of a 5' acrydite group that attaches the oligo to the hydrogel, a USER enzyme site, a spacer to reduce unwanted background reverse transcription noise, a sequencing adapter sequence, a 9-mer semi-randomized unique molecular identifier (UMI) and a poly-20TVN capture region.

A 6% acrylamide gel containing 1 µM of the oligo is casted on a silanized glass slide. 5 µL of the printing oligo mixture is covered with a 22 x 22 mm square cover slip and let polymerize at 25C for 30 mins. The cover slip is carefully lifted and excess non-polymerized oligo is rinsed away with MilliQ water.

Purchased fresh frozen sections of mouse olfactory bulb or mouse embryo E13 was thawed at room temperature for 5 mins and fixed for 10 mins in 4% formaldehyde (diluted 1:9 from 36.5-38.0 stock solution (Sigma-Aldrich) in 1× PBS). After fixation, the sections were rinsed with 1× PBS and 500 µL of pre-warmed 0.1% pepsin (Sigma-Aldrich) dissolved in 0.1M HCl was added on top of the tissue section. The section was incubated in a humidified chamber at 37 °C for 6-10 mins. (olfactory bulb: 6 mins and embryo: 10 mins). The solution was topped off and rinsed with 1× PBS. Excess liquid was dabbed dry with Kimwipe^{®}. 10 µL of reverse transcription solution was added onto the oligo hydrogel and the permeabilized section was stacked on top of the gel carefully avoiding air bubbles. The reverse transcription solution contained 1× First Strand buffer (Invitrogen), 5 mM DTT (Invitrogen), 500 µM dNTP mix, 50 ng/µL Actinomycin D (Sigma-Aldrich), 1% DMSO (NEB), 20U/µL Maxima H Minus Reverse Transcriptase (ThermoFisher Scientific), 1 µM iso-TS adapter and 2U/µL RNaseOUT (Invitrogen). The tissue section and oligo hydrogel was incubated in a humidified chamber at 42 °C overnight (15-16 hrs).

The template switching reaction can be enhanced the next day by removing the tissue section and adding the reverse transcription solution onto the oligo gel and perform a second round of reverse transcription at 42 °C for 1 hr.

### Iso-Template Switching (TS) adapter:

/5Me-isodC//iisodG//iMe-isodC/ ACA CTC TTT CCC TAC ACG ACG CTC TTC
CGA TCT rGrGrG (r for RNA base)

### Adding Spatial Information onto the cDNA Library Oligo Gel

The oligo array chip is stuck on a microscope slide with double sided tape. 35 µL of printing solution with 1× Thermopol Buffer (NEB), 0.2 µg/µL BSA, 200 µM dNTP mix, and 0.32U/µL Bst DNA polymerase, large fragment (NEB) was added on top of the oligo array chip. The glass slide with the oligo hydrogel containing cDNA is preheated at 94 °C for 3 mins in a slide PCR and directly cooled on an ice block. It is then stacked on top of the chip with the gel side facing the chip. The whole cassette is held together by a two 2" binder clip. The whole setup is incubated in a humidified container at 55 °C for 2 hrs. The two surfaces are separated by removing the binder clip and immersing the oligo hydrogel and oligo array chip, still stuck together, in 95C 0.3x SSC for 20 mins. The oligo hydrogel is released from the chip by carefully pushing the glass slide away from the chip.

The cDNA library attached to the hydrogel:

/5Acryd/UUUUU/iSp18/AGACGTGTGCTCTTCCGATCTWSNNWSNNVTTTTTT
TTTTTTTTTTTTTTVN – cDNA – AGATCGGAAGAGCGTCGTGTAGGGAAAGAGTGT
[30 mer_zipcode] AAGAGACGCGCTGACCCTGA

### Spatial Info Tagged cDNA Library Preparation and Sequencing

To determine how many PCR cycles is needed to amplify the sample, 1/5 volume of the sample is added to a qPCR mixture containing 1× KAPA HiFi Reaction Buffer, 0.3 mM dNTP mix, 0.5 µM sequencing adapter 1, 0.5 µM sequencing adapter 2, 1× EVA Green (Biotium) and 0.5U/reaction KAPA HiFi DNA Polymerase. After the number of cycles is determined for the sample, the remaining sample is amplified with the same conditions as the qPCR reaction. The amplified sample or sequencing library was purified using Agencourt AMPure XP beads with beads to sample ratio of 0.75: 1 and eluted in nuclease-free water (IDT). The concentration of the library was quantified using KAPA Library Quantification Kits (KAPA Biosystems) per the manufacturer's protocol. Libraries were diluted to 2nM and sequenced on the Illumina MiSeq platform using paired-end sequencing per the manufacturer's protocol.
Sequencing adapter 1:
   AAT GAT ACG GCG ACC ACC GAG ATC TTC AGG GTC AGC GCG TCT CTT
Sequencing adapter 2:

CAA GCA GAA GAC GGC ATA CGA GAT GTG ACT GGA GTT CAG ACG
TGT GCT CTT CCG ATC T

### Sequencing Alignment and Zipcode Decoding

The read containing the gene information was first aligned to the mouse genome using STAR. The reads that were aligned were extracted and the corresponding read that contains the zipcode was decoded for its positional x, y coordinate information. The number of reads aligned to each gene was counted with htseq-count. The reads that aligned to a coding transcript were extracted and whenever a read that contains a coding transcript and the corresponding read contains a decoded zipcode, the information is written to a new file that contains the combined gene expression and positional information.

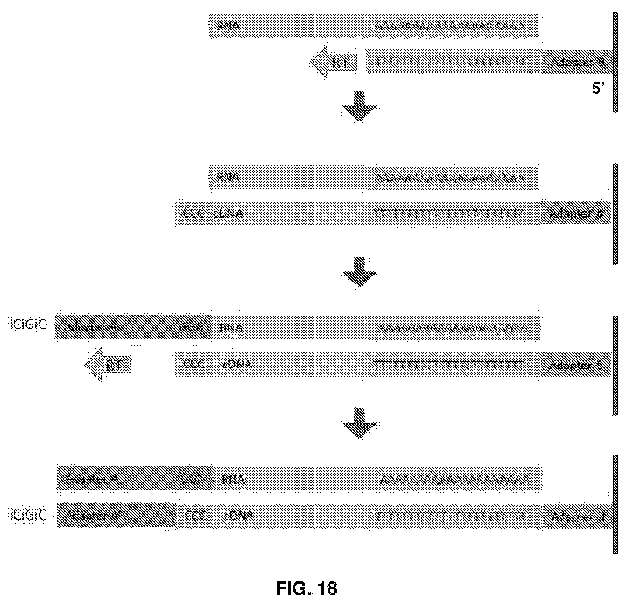

FIG. 18 shows the steps of capturing a RNA (comprising a poly-A tail) on poly-T oligo-containing gel and template switching reverse transcription to produce a cDNA sequence of the RNA. For example, a poly-T tail may be covalently attached to Adapter B. Adapter B may be covalently attached to a substrate surface, e.g., a gel matrix. The poly-T tail may hybridize to the poly-A tail of mRNA. The poly-T sequence may then be extended in a reverse transcription reaction catalyzed by a reverse transcriptase using the hybridized mRNA as a template to produce a cDNA molecule complementary to the mRNA. Terminal transferase activity of the reverse transcriptase can add additional based (e.g., poly-C) to the 3' end of the synthesized cDNA molecule. The additional based added to the cDNA molecule, e.g., poly-C, can further hybridize to a template-switching oligonucleotide comprising a complementary poly-G tail and another adapter (shown as "Adapter A" in FIG. 18). This hybridization can facilitate template switching so that a sequence complementary to the template-switching oligonucleotide can be incorporated into the strand comprising the cDNA molecule. In addition, incorporating non-natural nucleotides, such as an isocytosine (iC) or an isoguanosine 9iG) into the template-switching primer/oligonucleotide may reduce background and improve cDNA synthesis. For example, a sequence of iCiGiC can be added to one end of the template-switching primer/oligonucleotide, as shown in FIG. 18. The result is a double-stranded cDNA fragment immobilized on a gel matrix.

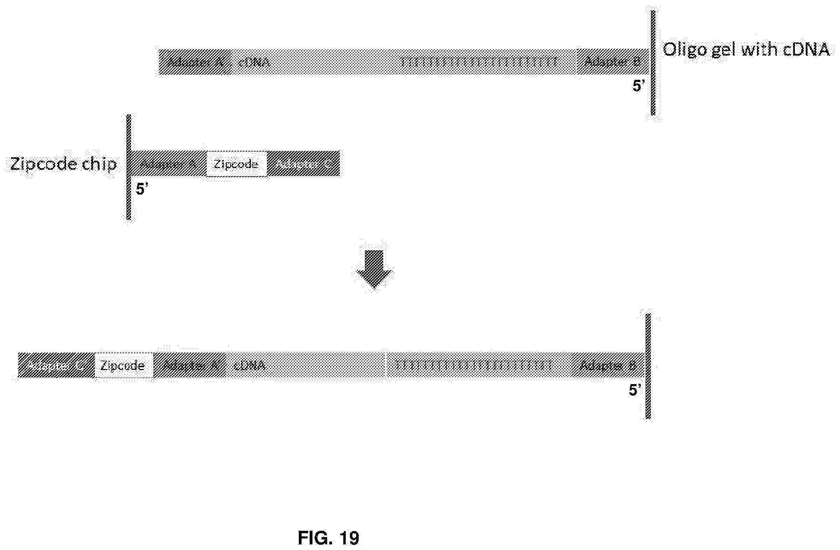

FIG. 19 shows the steps of denaturing of the cDNA sequence synthesized in FIG. 18, and using the cDNA-containing oligo gel matrix to print on a zipcode array chip. In some cases, the zipcode oligo on a zipcode chip comprises a sequence of "Adapter A" that is complementary to the sequence of "Adapter a" on the single-stranded cDNA on the oligo gel matrix. The zipcode oligo may also comprise a zipcode and another adapter sequence-"Adapter C." Further extension of the immobilized cDNA fragment using the zipcode oligo as a template may afford an immobilized cDNA fragment comprising a zipcode on the gel matrix, as shown in FIG. 19.

Then the zipcode-containing cDNA sequences may be subjected to PCR with Adapter B and Adapter C' to produce cDNA library for sequencing analysis.

### Example 4: Analysis of Protein Molecules

Protein and other biomolecules can be analyzed with labeled antibodies against the molecules of interest. The labels may contain a Tag sequence (comprising barcode sequence) indicating the identities of the target molecules. Linker sequences connecting with zipcodes and/or sequencing adaptors can be added.

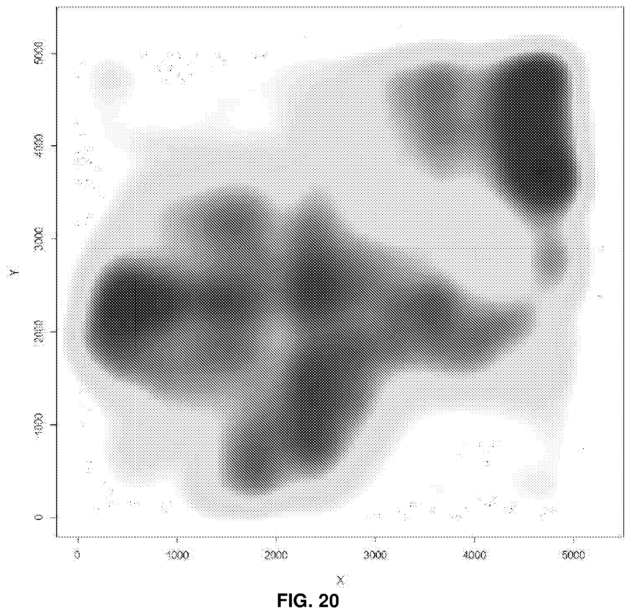

FIG. 20 shows T cell distribution in a mouse spleen. Two antibodies against T cells were labeled with tag sequence and eventually linked with zipcodes. After sequencing, the positions and tags were decoded and plotted using R's smoothScatter function.

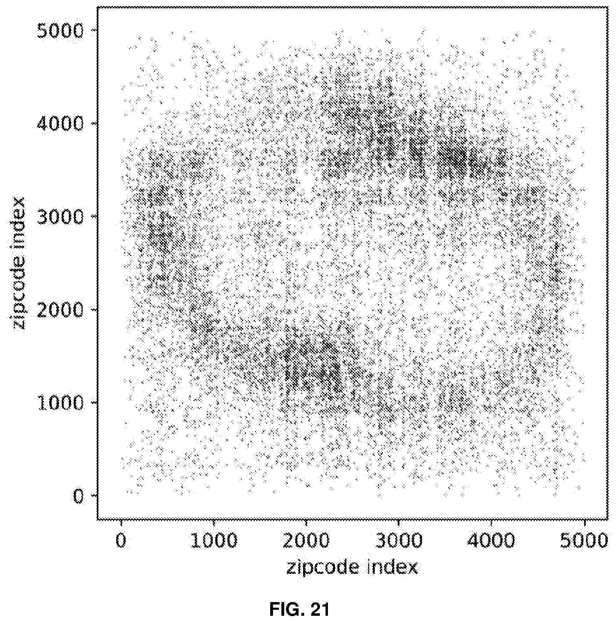

FIG. 21 shows spatial analysis of Microglial (Iba1-Tag) in mouse brain (unfiltered raw data plot).

The tag design used in FIGs. 20 and 21 may be as follows (the number after colon indicates the length of the nucleic acid sequence for each segment):
rcFC1:25-rcSP1:33-Zipcode:33-36-rcSC1:20-Tag:9-SP2:34-Index:6-FC2:24

### Example 5: DNA Stretching Directly on DNA Arrays and Zipcode Human Genome Sequencing

### Hybridization

1. Place Jacaranda chips (6Q0177) onto a microscope slide in a humidified chamber.
2. Dilute 1 µL of 100 mM Jacaranda onchip Y-adapter w/ FC1 with 29 µL 4x SSC buffer for a total volume of 30 µL.
3. Pipette the 30 µL mixture onto the chips and cover with a cover slip.
4. Hybridize at 35 °C for 4 hours.
5. Turn off oven and let it sit in oven for 1 hour.
6. Place at 4 °C overnight.

### Extension

7. Make sure the chamber containing the chips is cold by placing it on ice. All of the following steps for extension can be done with the chamber on ice.
8. Transfer chips to a 24 well plate with a separate well for each chip.
9. Equilibrate chips in 1 mL 1× NEB 2 Buffer for 5 minutes.
10. Remove chips from the buffer. Dry chips using Kimwipe^{®} by soaking buffer from the edge of the chip.
11. Adhere chips to a new microscope slide.
12. Prepare extension mixture as follows (NF-Water stands for nuclease free water):

| | 1× |
|---|---|
| NF-Water | 101 |
| NEB 2 Buffer | 12 |
| dNTP diluted 1:3 | 1 |
| Klenow large fragment | 6 |
| Final Volume | 120 µL |

13. Pipette 30 µL of the extension mixture onto each chip and cover the chips with a cover slip.
14. Place into a hybridization oven turned off for 30 minutes.
15. Remove from the oven, let extension proceed for 30 more minutes.

### Restriction Digest and Ligation

16. Equilibrate chips in 100 µL CutSmart^{®} Buffer (NEB) by pipetting directly onto chip. Let the mixture sit for 5 minutes.
17. Prepare restriction enzyme-DNA mixture as follows:

| | 1× |
|---|---|
| NF-Water | 13.15 |
| Human (male) genomic DNA (6.85 µL/µg) | 6.85 |
| CutSmart^{®} Buffer | 3 |
| ATP | 3 |
| Quick Ligase | 1 |
| Pmel | 1 |
| Alul/HaeIII/or HpyCH4V | 2 |
| Final Volume | 30 µL |

18. Add 30 µL of the restriction enzyme-DNA mixture onto the chip. Cover with a cover slip and leave overnight.

### PCR

19. Wash chips with 0.1 N NaOH for 5 minutes.
20. Fragment chips and place into PCR tube.
21. In PCR tube, add 50 µL 2× Kapa HiFi PCR mixture, 40 µL Water, and 10 µL Jacaranda onchip FC2 Y-adapter (Ref. 163486338).
22. Run PCR with the following settings:

| Temperature | Time | Hold/cycle |
|---|---|---|
| 98 °C | 3 minute | Hold |
| 98 °C | 1 minute | 12 cycles |
| 50 °C | 4 minute | |
| 72 °C | 30 seconds | |
| 72 °C | 2 minutes | Hold |
| 4 °C | Up to hours | Hold |

23. Take 18 µL of the PCR product from the previous step and add 1 µL Jacaranda FC1 top adapter (Ref. 162665178) and 1 µL FC2 bottom adapter (Ref. 162665179).
24. Run PCR with the following settings:

| Temperature | Time | Hold/cycle |
|---|---|---|
| 96 °C | 1 minute | Hold |
| 96 °C | 1 minute | 15 cycles |
| 67 °C | 30 seconds | |
| 72 °C | 1 minute | |
| 72 °C | 2 minutes | Hold |
| 4 °C | Up to hours | Hold |

A. Hybridize desired oligos. For Jacaranda oligos, make a 100 µM 50/50 mixture of desired oligos (i.e. 20 µL JACANCHOR Y3'P (Ref. 163486340) and 20 µL Jacaranda Anchor Y oligo (Ref. 162665174) for a total of 40 µL), then place on thermocycler with the following steps (1 run; no cycles):
   i. 94 °C - 1 min.
   ii. 55 °C - 10 min.
   iii. 25 °C - 10 min.
   iv. 4 °C - up to hours
B. Silanate iron-oxide wafers with the following steps:
   i. Combine 50 mL NF-water, 200 µL bind-silane, and 11 µL glacial acetic acid in a 50 mL conical flask.
   ii. Shake horizontally at 60 rpm for at least 15 minutes.
   iii. Place diced wafers with surface to be silanated facing up in a sealable plastic container
   iv. Pour silane mixture made above into the plastic container, making sure wafers are submerged.
   v. Shake at about 40 - 60 rpm for at least 1 hour.

### Casting the Gel onto Diced Wafers

1. Prepare desired concentration of oligo-acrylamide mixture as follows:
The final concentration of acrylamide in the mixture can be 6%.

| | 1× |
|---|---|
| 100 µM Hybridized Oligo | 1 |
| 40% acrylamide/bis solution | 15 |
| Nuclease free water | 84 |
| Final volume (µL) | 100 |

2. Prepare diluted APS and TEMED:
i. APS: 0.05 g in 1mL NF-water
ii. TEMED: 2 µL TEMED in 38 µL NF-water
Both solutions can be used for up to 2 days after they are made.
3. Set aside enough cover slips and a pair of tweezers for the number of gels needed.
4. Pipette 13.5 µL oligo-acrylamide mixture into separate PCR tubes.
5. Set a p20 pipette to 15 µL, and set a p2 pipette to 0.75 µL.
6. Quickly pipette 0.75 µL of the prepared APS into one PCR tube containing the oligo mixture, followed by 0.75 µL of the prepared TEMED into the same tube.
7. Use the pre-set p20 pipette to quickly transfer 15 µL of the mixture onto a silanized wafer. Immediately cover the wafer with a cover slip.
8. Repeat steps 7 and 8 for the remaining gels.
9. Allow from about 30 minutes to about 1 hour for polymerization.
10. Gently remove a cover slip with razor blade and proceed to stretching steps.

### Stretching Human Genomic DNA

11. Equilibrate gels in 1× 2-(N-morpholino)ethanesulfonic acid (MES) buffer 50 mM at pH 5.5 for 1 - 2 minutes (make enough buffer for gel to submerge in the buffer, for example, 300 µL)
12. Gently dab away excess moisture with Kimwipe^{®}, making sure not to touch the gel.
13. Each cuvette for stretching has a maximum volume of 1.5 mL. Clean with DI water and dry with compressed air.
14. Fill the cuvette with 1 µg of human (male) genomic DNA (Promega) suspended in 1 mL MES.
15. Dip gels for 1 hour.
16. Retract at 67 µm/s.
17. Place gels into plastic container humidified with water.

### Restriction Digest and Ligation

18. For each gel, make the 30 µL digestion mixture as follows:

| | 1× |
|---|---|
| NF-Water | 20 |
| CutSmart^{®} Buffer | 3 |
| ATP | 3 |
| Quick Ligase | 1 |
| Pmel | 1 |
| AluI/HaeIII/ or HpyCH4V | 2 |
| Final Volume | 30 µL |

19. Pipette 30 µL of the digestion mixture onto the gel and cover with a cover slip.
20. Incubate in room temperature (about 24 - 25°C) in the humidified chamber overnight before proceeding to the printing steps.

### Restriction Digest and Ligation without Stretching

a. Equilibrate gel in 100 µL 1× CutSmart^{®} Buffer for 5 minutes.
b. Discard buffer. Add the following enzyme-gDNA mixture:

| | 1X |
|---|---|
| Human (male) genomic DNA (6.85 µl/µg) | 6.85 |
| NF-Water | 13.15 |
| Cutsmart^{®} Buffer | 3 |
| ATP | 3 |
| Quick Ligase | 1 |
| PmeI | 1 |
| AluI/HaeIII/ or HpyCH4V | 2 |
| Final Volume | 30 µl |

c. Pipette 30 µL of the enzyme-gDNA mixture onto the gel and cover with a cover slip.
d. Let reaction proceed overnight at room temperature (about 24 - 25 °C) before proceeding to printing steps.

### Printing Jacaranda Chips onto Gel

21. Add 1 µL PmeI directly onto the digestion mixture and place the gels in humidified chamber at 37°C for 30 minutes.
22. Prepare 0.10 N NaOH solution, enough for 40 mL for each gel. Also prepare more than 20 mL 1× SSC Buffer for each gel. There are 2 NaOH washes, 20mL per wash. Use separate conical flask for each gel.
23. Place each gel in a separate conical flask containing 20 mL 0.10N NaOH solution. Shake by hand for 3 minutes.
24. Repeat the previous NaOH wash step in the another batch of fresh 0.1N NaOH solution.
25. Discard NaOH solutions. Equilibrate gels in least 100 µL 1× PNK Buffer per gel for 5 minutes.
26. Discard buffer. For each gel, add the following mixture of T4 PNK Kinase:

| | 1× |
|---|---|
| PNK Buffer | 2.5 |
| ATP | 2.5 |
| T4 PNK Kinase | 1 |
| NF-Water | 19 |
| Final Volume | 25 µL |

27. Cover with a cover slip.
28. Transfer gels back into humidified chamber and place in 37°C for 30 minutes.
29. Prepare and add 100 µL 1× Thermopol buffer for each gel and cover with a cover slip.
30. Place in 85 °C for 5 minutes to heat-inactivate the kinase and other enzymes.
31. Prepare printing solution as follows, making sure to add Bst just before assembling the chip cassette:

| | 1× |
|---|---|
| Bst (8 U/µL) [final .32 U/µL] | 4 |
| dNTP (19 mM) [final .2 mM] | 2 |
| 10x Thermopol Buffer | 10 |
| BSA (20 ng/µL) [final 0.2 µg] | 1 |
| Nuclease-free Water | 83 |
| Final volume | 100 µL |

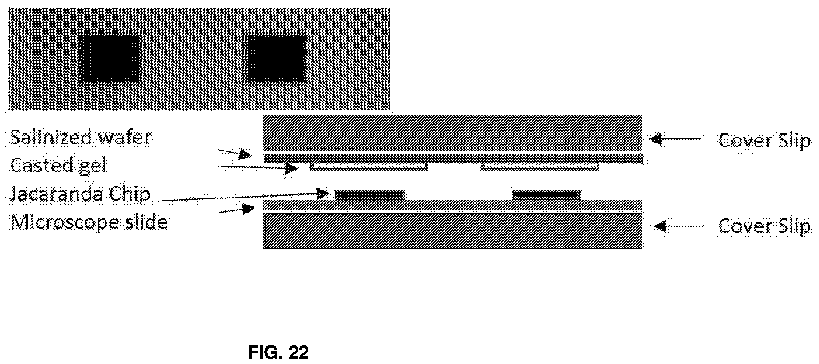

32. Remove cover slip, absorb extra moisture with Kimwipe^{®}, and use the 100 µL print solution to wet Jacaranda chip (6Q0177) and the gel.
33. Assemble cassettes. See FIG. 22 for an example. The top panel of FIG. 22 shows the top view of the Jacaranda Chip on a microscope slide, which lie at the bottom portion of the cassette. The bottom panel of FIG. 22 shows an example section view of the cassette. Briefly, double sided tapes are used to attach array chips (e.g., Jacaranda chip, the two darker blocks shown in the top panel of FIG. 22) to a microscope slide (the rectangle grey shape in the top panel of FIG. 22). Then, as shown in the bottom panel of FIG. 22, place a salinized wafer comprising two casted gel blocks on one side of the wafer above the microscope slide comprising the Jacaranda chips. Each of the casted gel on the salinized wafer substantially, if not completely, covers one of the Jacaranda chips on the microscope slide. To complete the cassette, place one cover slip on top of the salinized wafer and another cover slip below the microscope slide.
34. Place cassette into 55 °C oven and incubate for 3 hours.
35. Boil chip-gels for 15 minutes in a container of DI water heated by a water bath set to 100 °C, then separate the chip from the gel.

### Adding Flow Cell Adapters via PCR

36. Scrape gel with razor blade into a PCR tube.
In some cases, if the gel is dry, hydrate with some DI-water.
37. To each reaction, add the followings:
20 µL KAPA PCR mixture
18 µL NF-water
1 µL 1:3 diluted Jacaranda FC1 top adapter (Ref. 162665178)
1 µL 1:3 diluted Jacaranda FC2 bottom adapter (Ref. 162665179)

| Temperature | Time | Hold/cycle |
|---|---|---|
| 98°C | 1 minute | Hold |
| 98°C | 30 seconds | 20 cycles |
| 65°C | 1 minute | |
| 72°C | 1 minute | |
| 72°C | 3 minutes | Hold |
| 4°C | Up to hours | Hold |

### Gel Extraction

38. Run gel on PCR products. Use 10 µL product + 2 µL loading dye for each well.
39. Load 3 µL 100 bp ladder
40. Run at 100 V for at least 1 hour 15 minutes.
41. Visualize gel, cut from 300 bp to 1000 bp.
42. Digest gel in at least 6 mL dissolving buffer.
43. Extract with QIAGEN gel extraction kit (e.g., QIAquick Gel Extraction Kit). Elute with 25 µL NF-water at 55 °C.

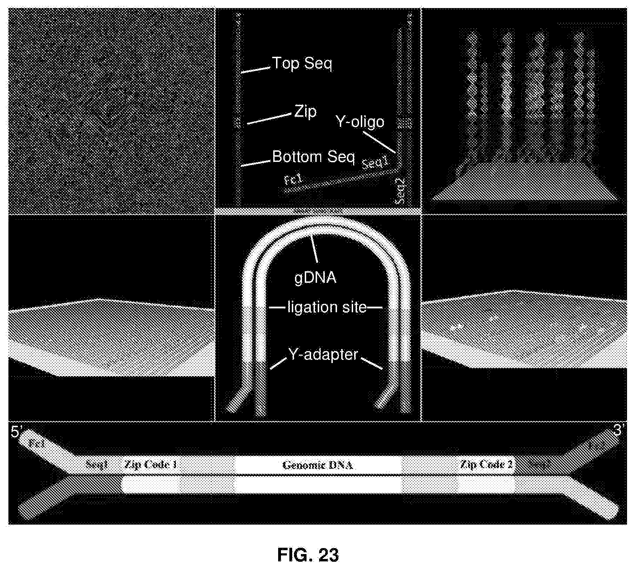

Turning now to FIG. 23, an overview of the on-chip approach for constructing genomic DNA sequencing libraries with a zipcode array is shown. The top left panel shows an example of a Centrillion zipcode array seen by fluorescence microscopy under 20× magnification. The array may comprise more than a million photolithographically patterned features, i.e., distinguishable positions. Each feature, for example, a square feature, may comprise a plurality of a unique DNA sequence, also known as the zipcode, which corresponds to the precise physical location of the specific feature on the zipcode array.

The top center panel of FIG. 23 shows an example zipcode sequence (on the left, single stranded, part of a Y-adapter) that is attached to the surface of each feature on the array substrate. Each zipcode sequence may comprise a bottom sequence (shown as Bottom Seq, may comprise a sequencing primer binding site (Seq2)) and a top sequence (shown as Top Seq) that are common to all features on the same zipcode array. The unique DNA sequence or zipcode (shown as Zip) may be between the bottom sequence and the top sequence.

A "Y-oligonucleotide" (single-stranded, shown as "Y-oligo" on the right side of the top center panel) may be allowed to hybridize with the immobilized zipcode sequence. The Y-oligonucleotide may comprise the flow cell adapter sequence Fc1 and a sequencing primer binding site (Seq1). Further, the Y-oligonucleotide may comprise a sequence complimentary to a top portion of the common bottom sequence of the zipcode, thereby allowing the Y-oligonucleotide to hybridize to the common bottom sequence of the zipcode sequences. Then a polymerase may extend the hybridized Y-oligonucleotide using the zipcode sequence as a template, starting from the free 3'-end of the Y-oligonucleotide hybridized to the common bottom sequence and through the zipcode and the top sequence parts of the zipcode sequence. In some cases after the polymerization, blunt double-stranded molecules with 5' phosphates may be formed on the surface of the zipcode array. The double-stranded molecule may comprise a copy of a zipcode sequence and a copy of the newly synthesized Y-oligonucleotide-based product of the polymerization reaction.

The top right panel of FIG. 23 shows that each feature on the zipcode array may comprise numerous double-stranded DNA molecules of identical sequence that have been patterned onto the zipcode array. Although the zipcode sequence may be unique to each feature, the zipcode may be common to all double-stranded DNA molecules within the same feature.

The middle left panel of FIG. 23 shows that genomic DNA can be stretched across the features (shown as squares) on the zipcode array, digested (e.g., by a restriction enzyme), and be prepared for covalent attachment (e.g., via enzyme ligations) to for double-stranded DNA fragment molecules on the zipcode array. The middle center panel shows an example design for a double-stranded DNA fragment molecule after ligation of a genomic DNA fragment to two surface-bound adapters. This design may employ a combination of vanishing and appearing restriction sites to allow spontaneous library assembly of a bound genomic DNA. As shown in the middle center panel, a genomic DNA fragment (shown as gDNA) may be ligated to two double-stranded Y-adapter molecules (shown as Y-adapter) on the array through the top sequence of the Y-adapter which comprises the zipcode to encode the physical location on the array on which this genomic fragment is located. The ligation sites between the genomic DNA fragment and the double-stranded Y-adapter molecules are shown as "ligation site" in the middle center panel of FIG. 23. Furthermore, as shown the Y-adapter may be covalently attached to the zipcode array surface via its 3'-end of its non-hybridized Y arm. See U.S. Patent Publication No. 20017/0044600 for a disclosure of library construction using Y-adapters and vanishing restriction sites, which is herein incorporated by reference in its entirety. The middle right panel of FIG. 23 shows a schematic view of genomic DNA fragments on top of a zipcode array. Each DNA fragment may have more than one zipcode sequence inserted in the middle or attached to the ends of the DNA fragment. Each zipcode sequence may encode positional information. The original genomic DNA may comprise several DNA fragments as shown. By piecing together the DNA fragments and relying on the zipcode sequence information, the full-length sequence of the corresponding genomic DNA may be obtained.

The bottom panel of FIG. 23 shows an example of an amplification product of the DNA fragments with zipcodes attached to both ends of the DNA fragments. For example, the double-stranded DNA fragment shown in the middle center panel can be denatured to form a single-stranded copy, which can be submitted to a low cycle number PCR reaction using Fc1-Seq1/Fc2-Seq2 primers to add the flow cell adapter sequences. This PCR reaction may amplify multiple copies of the DNA fragment in solution to form a library. The resulting library molecules may all have the same basic structure shown in the bottom panel of FIG. 23. Using this approach, each library molecule may contain zipcodes flanking the sequence of the genomic DNA fragment. A plurality of such library molecules may result in a fully formed library ready for next generation sequencing.

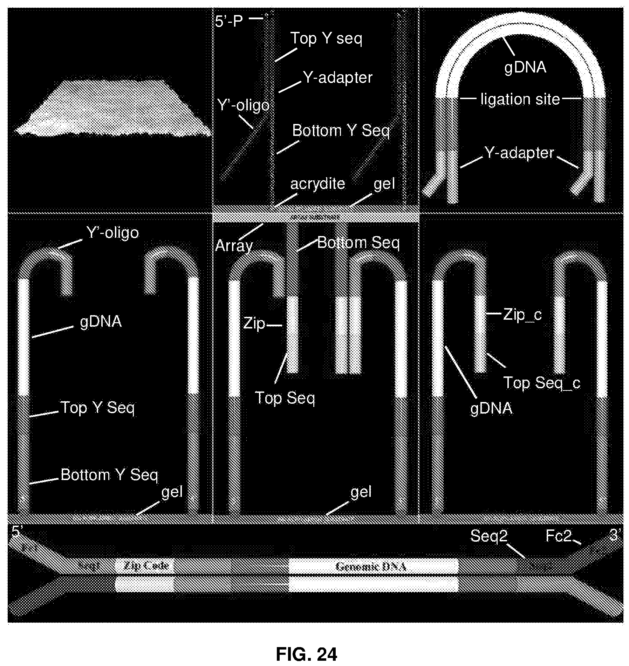

Turning now to FIG. 24, an overview of the zipcode printing approach for constructing genomic DNA sequencing libraries is shown. The top left panel of FIG. 24 shows that genomic DNA can be stretched out on top of a gel, such as, for example, a 6% polyacrylamide gel. The gel can comprise embedded Y-adapter oligonucleotides described below.

The top middle panel shows an example design of a single-stranded Y-adapter segment that is conjugated (i.e., covalently bound) to a 5' acrydite moiety (shown as "acrydite" in the top center panel of FIG. 24) which is also bound to the acrylamide gel surface. For example, during the gel formation process, this 5' acrydite moiety at the 5'-end of the bottom sequence of the single-stranded Y-adapter segment may allow the acrylamide monomers and the single-stranded Y-adapter segment to co-polymerize and form a hydrogel containing the single-stranded Y-adapter segments dispersed on and throughout the acrylamide gel matrix. The bottom sequence segment of the single-stranded Y-adapter segment (shown as "Bottom Y Seq" in the top middle panel) may serve as a sequencing primer as well as the binding site of the Fc2 primer when the library of DNA fragments is liberated from the acrylamide matrix. The top sequence segment of the single-stranded Y-adapter segment (shown as "Top Y Seq" in the top middle panel) may contain half of the Pmel restriction site. Then another Y-oligonucleotide (shown as "Y'-oligo" in the top center panel) may be allowed to hybridize with the immobilized single-stranded Y-adapter segment to complete the formation of the Y-adapter on the surface of the acrylamide gel matrix. The 5'-end of the Y-oligonucleotide may comprise a free phosphate (shown as "5'-P" in the top center panel) either before or after the hybridization. As shown, this Y-adapter may not comprise a zipcode sequence. The construction of acrylamide gel matrix with immobilized Y-adapter can be accomplished in many ways. For example, instead of using the single-stranded Y-adapter segments during polymerization, the hybridized Y-adapters (comprising both the single-stranded Y-adapter segment and the Y-oligonucleotide) may be used in the polymerization step to form the acrylamide gel matrix with immobilized Y-adapters attached to the surface. The phosphate on the 5' end of the Y-adaptor can be introduced before or after the polymerization step.

Digestion and ligation of stretched genomic DNA, using vanishing and appearing restriction sites methodology disclosed in U.S. Patent Publication No. 20017/0044600, may generate Y-adapters covalently attached to genomic DNA fragments, as shown in the top right panel of FIG. 24. The DNA fragment is shown as "gDNA" in the top right panel. The ligation site is between the DNA fragment and the Y-adapters in the top right panel.

A wash with NaOH followed by a heat denaturation step may separate the ligated strands (see the top right panel) from each other and prepare them for hybridization to a zipcode array, such as, for example, the one described in the top middle panel of FIG. 23. The middle left panel of FIG. 24 shows two single stranded DNA sequences immobilized to the acrylamide gel matrix. For example, the immobilized DNA sequence on the left comprises, sequentially from the surface of the acrylamide gel matrix, a bottom sequence (shown as "Bottom Y Seq" in the middle left panel), a top sequence (shown as "Top Y Seq" in the middle left panel), a single-stranded genomic DNA fragment (shown as "gDNA" in the middle left panel) and a Y-oligonucleotide (shown as "Y'-oligo" in the middle left panel).

Then, a zipcode array, such as, for example, the one described in the top middle panel of FIG. 23 can be placed in contact with the acrylamide gel matrix obtained above. For example, as shown in the middle center panel of FIG. 24, the zipcode array (shown as "Array" in the middle center panel of FIG. 24) may comprise a plurality of features, each of which comprises zipcode sequences to encode for the spatial information of the zipcode sequence on the zipcode array. Each single-stranded zipcode sequence may comprise a bottom sequence (shown as "Bottom Seq" in the middle center panel of FIG. 24) and a top sequence (shown as "Top Seq" in the middle center panel of FIG. 24) that are common to all features on the same zipcode array; and a unique DNA sequence or zipcode (shown as "Zip" in the middle center panel of FIG. 24) between the bottom sequence and the top sequence of the zipcode sequence). The surface of the acrylamide gel matrix which comprises immobilized DNA sequences (comprising Y-adapters and DNA fragments) may be placed in contact with the surface of the zipcode array which comprises zipcode sequences to allow hybridization reactions. As shown in the middle center panel, a portion of the Y-oligonucleotide attached to the DNA sequence may be complementary to a portion of the bottom sequence of the zipcode sequence. After hybridization of the DNA sequences on acrylamide gel matrix to the bottom sequence of the zipcode sequence, a polymerase can be added, together with other required reagents for extension reactions, to extend the hybridized Y-oligonucleotide through the zipcode sequence and top sequence of the single-stranded zipcode sequence on the zipcode array.

After the extension is complete, separation of the acrylamide gel matrix from the zipcode array may result in immobilized oligonucleotides on the acrylamide gel matrix, as shown in the middle right panel of FIG. 24. For example, an immobilized oligonucleotide may comprise a genomic DNA fragment (shown as "gDNA" in the middle right panel of FIG. 24) and a zipcode (shown as "Zip_c" in the middle right panel of FIG. 24) to encode the spatial positional information. In addition, a top sequence (shown as "Top Seq_c" in the middle right panel of FIG. 24) may be attached to each genomic DNA fragment as well. This top sequence may serves as a sequencing primer as well as a primer binding site to introduce the Fc1 sequence.

Liberation of the DNA fragments library from the acrylamide gel matrix using Fc1-Seq1/Fc2-Seq2 primers may give a library with the general structure shown in the bottom panel of FIG. 24. Using this approach one zipcode may be attached to each genomic DNA fragment.

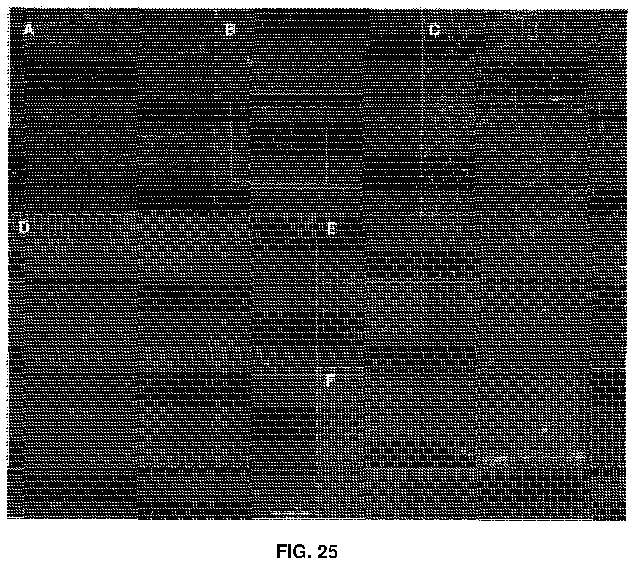

Turning now to FIG. 25, heavy molecular weight molecules of human genomic DNA molecules can be deposited in an elongated conformation on a variety of surfaces. Panel A of FIG. 25 shows Human Genomic DNA molecules were stained with YOYO-1 Iodide at a ratio of 1 molecule dye/5bp DNA; stretched on a 500 nm oxide coated silica wafer covered in a monolayer of 10-undecenyl trichlorosilane. Panel B of FIG. 25 shows YOYO-1 stained human genomic DNA molecules were stretched on a 6% polyacrylamide gel matrix. The 6% polyacrylamide gel matrix comprises 1 µM Y-adapter oligonucleotides covalently bonded to the gel matrix through a 5' acrydite moiety at one end of the Y-adapter oligonucleotides. After stretching the DNA, the gel max was soaked overnight in 2 ml T4 Ligase buffer with gentle shaking at room temperature and imaged the next day. Panel C of FIG. 25 shows an inset from Panel B of FIG. 25. Panel C shows a magnified region of the acrylamide gel matrix from Panel B. High molecular weight genomic DNA fragments may be readily visible in Panel C and may appear to have maintained their elongated conformation despite the overnight shaking in the Ligase buffer. Panel D of FIG. 25 shows a large microscope scan that shows YOYO-1 stained human genomic DNA stretched directly on a Centrillion Array. Patterned DNA features are readily apparent in the background in Panel D. Panel E is an inset from Panel D. Panel E shows a magnified region of the Centrillion Array shown in Panel D. Numerous molecules of genomic DNA greater than 200 kb in size are readily visible in Panel E. Panel F of FIG. 25 shows a high magnification of a Centrillion Array which shows a single molecule of genomic DNA that may be hundreds of kilobases long, stretched out across more than two dozen patterned DNA features on the array. All images in FIG. 25 were taken on a Keyence BXT inverted microscope. All substrates were soaked for one hour in a stretching cuvette in MES buffer (50 mM at pH 5.5). For Panels A-E, the biological sample may contain 50 pg/µL genomic DNA. For Panel F the biological sample may contain 15 pg/µL genomic DNA. The genomic DNA samples were retracted from solution at a rate of 67 m/sec.

The description above shows various examples and embodiments. A zipcode array can be used to analyze a variety of molecules including DNA, RNA and protein molecules in 2D formats. The zipcode array can be used to analyze molecules in 3D formats as well. For example, a tissue sample may be sliced vertically into a stack of sheets, and each sheet of tissue sample may be associated with an index number denoting the relative position to other sheets of tissue sample. Each sheet of tissue sample may be analyzed for a variety of molecules including DNA, RNA and protein molecules in 2D formats. However, the zipcode arrays used may comprise zipcode sequences comprising, in addition to the x and y coordinates, a z coordinates, such that, for tissue analysis, 3D analysis can use the z coordinates for each sheet of tissue sample to provide assemble 3D information of the variety of molecules.

The methods, kits and devices describe in this disclosure may analyze a variety of molecules in biological samples or material samples. In cases of genome analysis, the 2D information may be used to decode the arrangement of subsequences of a long DNA sequence. In other cases, the zipcode arrays may be used to decode positional information of molecules at a cellular resolution. For example, zipcode arrays may be at 2 µM resolution containing more than 25 million zipcodes. Higher resolution can be achieved by reducing the feature size to nm range using higher resolution oligonucleotide array synthesis methods.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

### Aspects of the Invention

1. A method for detecting spatial distribution of a plurality of target molecules within a biological sample, comprising:
   (a) providing a substrate comprising a plurality of distinct locations, each of the plurality of distinct locations comprises two or more coordinates;
   (b) attaching a plurality of zipcodes to the plurality of distinct locations;
   (c) contacting a biological sample comprising a plurality of target molecules with the substrate, thereby generating a plurality of report molecules, each report molecule comprising:
      (i) a fragment of a first target molecule of the plurality of target molecules, or a barcode indicating the presence of the first target molecule; and
      (ii) a first zipcode of the plurality of zipcodes, wherein the first zipcode encodes coordinates of contact on the substrate for the fragment of the first target molecule or the barcode; and
   (d) sequencing the plurality of report molecules and determining the coordinates of contact, thereby determining the spatial distribution of the plurality of target molecules within the biological sample.
2. The method of aspect 1, wherein the plurality of target molecules are deoxyribonucleic acids (DNA), ribonucleic acids (RNA), complementary deoxyribonucleic acids (cDNA), proteins, carbohydrates, lipids, natural products, antigens, metabolites, peptides, aptamer, cells, or binding partners thereof.
3. The method of aspect 2, wherein the binding partners are antibodies, aptamers, or synthetic antibody mimics.
4. The method of aspect 2, wherein each binding partner comprises another barcode encoding for the target molecule each binding partner binds to or recognizes.
5. The method of aspect 1, wherein each zipcode comprises (i) a bottom adapter attached to a distinct location, (ii) a coordinate zipcode attached to the bottom adapter; and (iii) a top adapter attached to the coordinate zipcode.
6. The method of aspect 5, wherein the coordinate zipcode encodes the two or more coordinates of the distinct location the coordinate zipcode is attached to.
7. The method of aspect 1, wherein each zipcode comprises (i) a bottom adapter attached to a distinct location, (ii) a lower zipcode attached to the bottom adapter; (iii) a separator sequence attached to the lower zipcode; (iv) an upper zipcode attached to the separator sequence; and (v) a top adapter attached to the upper zipcode.
8. The method of aspect 7, wherein the lower zipcode encodes a first coordinate and the upper zipcode encodes a second coordinate, and wherein the two or more coordinates comprises the first coordinate and the second coordinate for the distinct location.
9. The method of aspect 1, wherein the report molecule is deoxyribonucleic acids (DNA) or derivatives thereof.
10. The method of aspect 1, wherein the biological sample is a tissue section, a derivative of the tissue section, a transfer of the tissue section, or a derivative of the transfer of the tissue section.
11. A method for detecting spatial distribution of a plurality of target molecules in a biological sample, comprising:
   (a) providing a substrate having a plurality of distinct locations, each distinct location comprising a first and second coordinates ;
   (b) attaching a plurality of zipcodes to each distinct location; thereby encoding the first and second coordinates by the plurality of zipcodes attached to each distinct location;
   (c) contacting a biological sample comprising a plurality of target molecules with a plurality of binding partners, wherein at least a fraction of the plurality of binding partners bind to or recognize at least a fraction of the plurality of target molecules to form a plurality of first tagged complexes;
   (d) placing the plurality of first tagged complexes on the substrate, thereby allowing the binding partners in the plurality of first tagged complexes to bind to or recognize at least a fraction of the plurality of zipcodes to form a plurality of second tagged complexes;
   (e) generating a plurality of report molecules based on the plurality of second tagged complexes; wherein each report molecule encodes for a selected binding partner and a selected zipcode in one of the plurality of second tagged complexes; and
   (f) sequencing the plurality of report molecules and determining the first and second coordinates and the binding partner for each report molecule; thereby determining the spatial distribution of the plurality of target molecules within the biological sample.
12. The method of aspect 11, wherein the plurality of target molecules are deoxyribonucleic acids (DNA), ribonucleic acids (RNA), complementary deoxyribonucleic acids (cDNA), proteins, carbohydrates, lipids, natural products, antigens, metabolites, peptides, aptamers, or cells.
13. The method of aspect 11, wherein the plurality of binding partners are antibodies, aptamers, or synthetic antibody mimics.
14. The method of aspect 11, wherein each of the plurality of binding partners comprises a barcode encoding for the target molecule it binds to or recognizes.
15. The method of aspect 11, wherein in (d) the forming the plurality of second tagged complexes is ligating by a ligase, a gap filling, annealing, or hybridizing.
16. The method of aspect 11, wherein each zipcode comprises (i) a first bottom adapter attached to the distinct location, (ii) a first coordinate zipcode attached to the first bottom adapter; and (iii) a first top adapter attached to the first coordinate zipcode.
17. The method of aspect 16, wherein the first top adapter is a primer that enables tagging the binding partners.
18. The method of aspect 16, wherein the first bottom adapter is a sequencing adaptor for sequencing library.
19. The method of aspect 16, wherein the first coordinate zipcode encodes the first and second coordinates.
20. The method of aspect 11, wherein each zipcode comprises (i) a first bottom adapter attached to the distinct location, (ii) a first lower zipcode attached to the first bottom adapter; (iii) a first separator sequence attached to the first lower zipcode; (iv) a first upper zipcode attached to the first separator sequence; and (v) a first top adapter attached to the first upper zipcode.
21. The method of aspect 20, wherein the first lower zipcode encodes the first coordinate and the first upper zipcode encodes the second coordinate.
22. The method of aspect 20, wherein the first separator sequence comprises a sequence selected from GGG, CCC and TT.
23. The method of aspect 20, wherein the first lower zipcode comprises from 5 to 24 bases.
24. The method of aspect 20, wherein the first lower zipcode comprises no more than 16 bases.
25. The method of aspect 20, wherein the first upper zipcode comprises from 5 to 24 bases.
26. The method of aspect 20, wherein the first upper zipcode comprises no more than 16 bases.
27. The method of aspect 11, wherein different zipcodes attached to different distinct locations have an edit distance of 4.
28. The method of aspect 11, wherein different zipcodes attached to different distinct locations have a long-range minimum edit distance of 5.
29. The method of aspect 11, wherein the biological sample is a tissue section or a transfer of a tissue section.
30. The method of aspect 11, wherein each report molecule is a deoxyribonucleic acid (DNA).
31. A method for detecting spatial distribution of biomolecule expression, comprising:
   (a) providing a substrate having a plurality of distinct locations, each distinct location comprising a first and second coordinates;
   (b) attaching a plurality of zipcodes to each distinct location; thereby encoding the first and second coordinates by the zipcodes;
   (c) contacting a biological sample comprising a plurality of biomolecules with the plurality of zipcodes, thereby attaching at least a fraction of the plurality of zipcodes with at least a fraction of the plurality of biomolecules or fragments thereof, or at least a fraction of copies of the plurality of biomolecules or fragments thereof, and generating a plurality of tagged molecules; and
   (d) sequencing the plurality of tagged molecules and determining the first and second coordinates for at least the fraction of the plurality of biomolecules; thereby determining the spatial distribution of the plurality of biomolecules within the biological sample.
32. The method of aspect 31, wherein the plurality of biomolecules are deoxyribonucleic acid (DNA).
33. The method of aspect 31, wherein the plurality of biomolecules are complementary deoxyribonucleic acid (cDNA) of ribonucleic acid (RNA).
34. The method of aspect 33, wherein the RNA is messenger RNA (mRNA).
35. The method of aspect 34, further comprising, prior to (c), reverse transcribing the mRNA to the complementary DNA (cDNA).
36. The method of aspect 31, wherein each zipcode comprises (i) a first bottom adapter attached to the distinct location, (ii) a first coordinate zipcode attached to the first bottom adapter; and (iii) a first top adapter attached to the first coordinate zipcode.
37. The method of aspect 36, wherein the first top adapter is a primer enabling tagging biomolecules.
38. The method of aspect 36, wherein the first bottom adapter is a sequencing adaptor for sequencing library.
39. The method of aspect 36, wherein the first coordinate zipcode encodes the first and second coordinates.
40. The method of aspect 31, wherein each zipcode comprises (i) a first bottom adapter attached to the distinct location, (ii) a first lower zipcode attached to the first bottom adapter; (iii) a first separator sequence attached to the first lower zipcode; (iv) a first upper zipcode attached to the first separator sequence; and (v) a first top adapter attached to the first upper zipcode.
41. The method of aspect 40, wherein the first lower zipcode encodes the first coordinate and the first upper zipcode encodes the second coordinate.
42. The method of aspect 40, wherein the first separator sequence comprises a sequence selected from GGG, CCC and TT.
43. The method of aspect 40, wherein the first lower zipcode comprises from 5 to 24 bases.
44. The method of aspect 40, wherein the first lower zipcode comprises no more than 16 bases.
45. The method of aspect 40, wherein the first upper zipcode comprises from 5 to 24 bases.
46. The method of aspect 40, wherein the first upper zipcode comprises no more than 16 bases.
47. The method of aspect 31, wherein different zipcodes attached to different distinct locations have an edit distance of 4.
48. The method of aspect 31, wherein different zipcodes attached to different distinct locations have a long-range minimum edit distance of 5.
49. The method of aspect 31, wherein the biological sample is a tissue section or a transfer of a tissue section.
50. The method of aspect 31, wherein the attaching in (c) comprises ligating or annealing.
51. A zipcode array, comprising:
   (a) a first location; and
   (b) a plurality of first zipcodes attached to the first location, wherein each first zipcode comprises (i) a first bottom adapter attached to the first location, (ii) a first lower zipcode attached to the first bottom adapter; (iii) a first separator sequence attached to the first lower zipcode; (iv) a first upper zipcode attached to the first separator sequence; and (v) a first top adapter attached to the first upper zipcode.
52. The zipcode array of aspect 51, wherein the first location comprises a first coordinate and a second coordinate.
53. The zipcode array of aspect 52, wherein the first lower zipcode encodes the first coordinate, and wherein the first upper zipcode encodes the second coordinate.
54. The zipcode array of aspect 51, wherein the first separator sequence comprises a sequence selected from GGG, CCC and TT.
55. The zipcode array of aspect 51, wherein the first lower zipcode comprises from 5 to 24 bases.
56. The zipcode array of aspect 51, wherein the first lower zipcode comprises no more than 16 bases.
57. The zipcode array of aspect 51, wherein the first upper zipcode comprises from 5 to 24 bases.
58. The zipcode array of aspect 51, wherein the first upper zipcode comprises no more than 16 bases.
59. The zipcode array of aspect 51, further comprising:
   (c) a second location;
   (d) a plurality of second zipcodes attached to the second location, wherein each second zipcode comprises (i) a second bottom adapter attached to the second location, (ii) a second lower zipcode attached to the second bottom adapter; (iii) a second separator sequence attached to the second lower zipcode; (iv) a second upper zipcode attached to the second separator sequence; and (v) a second top adapter attached to the second upper zipcode.
60. The zipcode array of aspect 59, wherein the second location comprises a third coordinate and a fourth coordinate.
61. The zipcode array of aspect 60, wherein the second lower zipcode encodes the third coordinate and the second upper zipcode encodes the third coordinate.
62. The zipcode array of aspect 59, wherein the second separator sequence comprises a sequence selected from GGG, CCC and TT.
63. The zipcode array of aspect 59, wherein the second lower zipcode comprises from 5 to 24 bases.
64. The zipcode array of aspect 59, wherein the second lower zipcode comprises no more than 16 bases.
65. The zipcode array of aspect 59, wherein the second upper zipcode comprises from 5 to 24 bases.
66. The zipcode array of aspect 59, wherein the second upper zipcode comprises no more than 16 bases.
67. The zipcode array of aspect 59, wherein the first and second locations are adjacent, and wherein both the first and second lower zipcodes pair and the first and second upper zipcodes pair have an edit distance of 4.
68. The zipcode array of aspect 59, wherein the first and second locations are not adjacent, and wherein the first and second lower zipcodes have a long-range minimum edit distance of at least 5.
69. The zipcode array of aspect 59, wherein the first and second locations are not adjacent, and wherein the first and second upper zipcodes have a long-range minimum edit distance of 5.
70. The zipcode array of aspect 51, wherein the first location is no more than 5 µm in length.
71. The zipcode array of aspect 70, wherein the first location is no more than 2 µm in length.
72. The zipcode array of aspect 51, further comprising more than 1 million first locations, wherein each first location is different from another.
73. The zipcode array of aspect 51, wherein the first bottom adaptor is a sequencing adaptor.
74. The zipcode array of aspect 51, wherein the first top adaptor is a primer.
75. A zipcode array, comprising:
   (a) a first location;
   (b) a second location;
   (c) a plurality of first zipcodes attached to the first location, wherein each first zipcode comprises (i) a first bottom adapter attached to the first location, (ii) a first coordinate zipcode attached to the first bottom adapter; and (iii) a first top adapter attached to the first coordinate zipcode; and
   (d) a plurality of second zipcodes attached to the second location, wherein each second zipcode comprises (i) a second bottom adapter attached to the first location, (ii) a second coordinate zipcode attached to the second bottom adapter; and (iii) a second top adapter attached to the second coordinate zipcode.
76. The zipcode array of aspect 75, wherein the first location comprises a first coordinate and a second coordinate.
77. The zipcode array of aspect 76, wherein the first coordinate zipcode encodes the first coordinate and the second coordinate.
78. The zipcode array of aspect 75, wherein the first coordinate zipcode comprises from 6 to 48 bases.
79. The zipcode array of aspect 75, wherein the first coordinate zipcode comprises no more than 32 bases.
80. The zipcode array of aspect 75, wherein the second location comprises a third coordinate and a fourth coordinate.
81. The zipcode array of aspect 80, wherein the second coordinate zipcode encodes the third coordinate and the fourth coordinate.
82. The zipcode array of aspect 75, wherein the second coordinate zipcode comprises from 6 to 48 bases.
83. The zipcode array of aspect 75, wherein the second coordinate zipcode comprises no more than 32 bases.
84. The zipcode array of aspect 75, wherein the first location is no more than 5 µm in length.
85. The zipcode array of aspect 84, wherein the first location is no more than 2 µm in length.
86. The zipcode array of aspect 75, further comprising more than 1 million locations including the first and second locations, wherein each location of the more than 1 million locations is distinguishable from another.
87. The zipcode array of aspect 75, wherein the first bottom adaptor is a sequencing adaptor.
88. The zipcode array of aspect 75, wherein the first top adaptor is a primer.
89. A method for detecting spatial distribution of a plurality of ribonucleic acid molecules in a biological sample, comprising:
   (a) contacting a first surface comprising a plurality of first oligonucleotides with a biological sample comprising a plurality of ribonucleic acid molecules;
   (b) extending a fraction of the plurality of first oligonucleotides by a transcriptase using the plurality of ribonucleic acid molecules as templates, thereby generating a plurality of second oligonucleotides, each of the plurality of second oligonucleotides comprising a fragment of complementary DNA (cDNA) of one of the plurality of ribonucleic acid molecules;
   (c) contacting a zipcode array comprising a plurality of zipcode oligonucleotides with the plurality of second oligonucleotides in the presence of a polymerase, thereby extending the plurality of second oligonucleotides and generating a plurality of third oligonucleotides, each of the plurality of third oligonucleotides comprising one of the plurality of second oligonucleotides and a complementary sequence of one of the plurality of zipcode oligonucleotides;
   (d) separating the first surface comprising the plurality of third oligonucleotides from the zipcode array; and
   (e) sequencing the plurality of third oligonucleotides; thereby determining the spatial distribution of the plurality of ribonucleic acid molecules within the biological sample.
90. The method of aspect 89, wherein the extending in (c) further comprises a template switching reaction.
91. The method of aspect 89, further comprising, in (b) after transcription, denaturing second oligonucleotides from ribonucleic acid molecule templates.
92. The method of aspect 89, wherein the first surface is a gel matrix.
93. The method of aspect 89, wherein the zipcode array comprises a plurality of distinct locations, and wherein each distinct location comprises a first coordinate and a second coordinate.
94. The method of aspect 93, wherein a plurality of first zipcode oligonucleotides attached to a first distinct location of the plurality of distinct locations encode the first coordinate of the first distinct location and the second coordinate of the first distinct location.
95. The method of aspect 94, wherein each first zipcode oligonucleotide comprises (i) a bottom adapter attached to the first distinct location, (ii) a coordinate zipcode attached to the bottom adapter; and (iii) a top adapter attached to the coordinate zipcode.
96. The method of aspect 94, wherein each first zipcode oligonucleotide comprises (i) a bottom adapter attached to the first distinct location, (ii) a lower zipcode attached to the bottom adapter; (iii) a separator sequence attached to the lower zipcode; (iv) an upper zipcode attached to the separator sequence; and (v) a top adapter attached to the upper zipcode.
97. The method of aspect 96, wherein the lower zipcode encodes the first coordinate of the first distinct location and the upper zipcode encodes the second coordinate of the first distinct location.
98. The method of aspect 89, wherein the biological sample is a tissue section, a derivative of the tissue section, a transfer of the tissue section, or a derivative of the transfer of the tissue section.
99. The method of aspect 89, wherein at least two second oligonucleotides of the plurality of second oligonucleotides comprise different fragments of complementary DNA (cDNA) sequence(s).
100. The method of aspect 89, wherein orientation of the plurality of zipcode oligonucleotides on the zipcode array is from 5' to 3'.

## Claims

1. A zipcode array, comprising:
(a) a first location; and
(b) a plurality of first zipcodes attached to the first location, wherein each first zipcode comprises (i) a first bottom adapter attached to the first location, (ii) a first lower zipcode attached to the first bottom adapter; (iii) a first separator sequence attached to the first lower zipcode; (iv) a first upper zipcode attached to the first separator sequence; and (v) a first top adapter attached to the first upper zipcode.

2. The zipcode array of claim 1, wherein the first location comprises a first coordinate and a second coordinate, optionally wherein the first lower zipcode encodes the first coordinate, and wherein the first upper zipcode encodes the second coordinate.

3. The zipcode array of claim 1, wherein the first separator sequence comprises a sequence selected from GGG, CCC and TT.

4. The zipcode array of claim 1, wherein:
(i) the first lower zipcode comprises from 5 to 24 bases; or
(ii) the first lower zipcode comprises no more than 16 bases; or
(iii) the first upper zipcode comprises from 5 to 24 bases; or
(iv) the first upper zipcode comprises no more than 16 bases.

5. The zipcode array of claim 1, further comprising:
(c) a second location;
(d) a plurality of second zipcodes attached to the second location, wherein each second zipcode comprises (i) a second bottom adapter attached to the second location, (ii) a second lower zipcode attached to the second bottom adapter; (iii) a second separator sequence attached to the second lower zipcode; (iv) a second upper zipcode attached to the second separator sequence; and (v) a second top adapter attached to the second upper zipcode.

6. The zipcode array of claim 5, wherein the second location comprises a third coordinate and a fourth coordinate, optionally wherein the second lower zipcode encodes the third coordinate and the second upper zipcode encodes the third coordinate.

7. The zipcode array of claim 5, wherein the second separator sequence comprises a sequence selected from GGG, CCC and TT.

8. The zipcode array of claim 5, wherein:
(i) the second lower zipcode comprises from 5 to 24 bases; or
(ii) the second lower zipcode comprises no more than 16 bases; or
(iii) the second upper zipcode comprises from 5 to 24 bases; or
(iv) the second upper zipcode comprises no more than 16 bases.

9. The zipcode array of claim 5, wherein:
(i) the first and second locations are adjacent, and wherein both the first and second lower zipcodes pair and the first and second upper zipcodes pair have an edit distance of 4; or
(ii) the first and second locations are not adjacent, and wherein the first and second lower zipcodes have a long-range minimum edit distance of at least 5; or
(iii) the first and second locations are not adjacent, and wherein the first and second upper zipcodes have a long-range minimum edit distance of 5.

10. The zipcode array of claim 1, wherein:
(i) the first location is no more than 5 µm in length, optionally wherein the first location is no more than 2 µm in length; or
(ii) the zipcode array further comprises more than 1 million first locations, wherein each first location is different from another; or
(iii) the first bottom adaptor is a sequencing adaptor; or
(iv) the first top adaptor is a primer.

11. A zipcode array, comprising:
(a) a first location;
(b) a second location;
(c) a plurality of first zipcodes attached to the first location, wherein each first zipcode comprises (i) a first bottom adapter attached to the first location, (ii) a first coordinate zipcode attached to the first bottom adapter; and (iii) a first top adapter attached to the first coordinate zipcode; and
(d) a plurality of second zipcodes attached to the second location, wherein each second zipcode comprises (i) a second bottom adapter attached to the first location, (ii) a second coordinate zipcode attached to the second bottom adapter; and (iii) a second top adapter attached to the second coordinate zipcode.

12. The zipcode array of claim 11, wherein:
(i) the first location comprises a first coordinate and a second coordinate, optionally wherein the first coordinate zipcode encodes the first coordinate and the second coordinate; or
(ii) the first coordinate zipcode comprises from 6 to 48 bases; or
(iii) the first coordinate zipcode comprises no more than 32 bases.

13. The zipcode array of claim 11, wherein:
(i) the second location comprises a third coordinate and a fourth coordinate, optionally wherein the second coordinate zipcode encodes the third coordinate and the fourth coordinate; or
(ii) the second coordinate zipcode comprises from 6 to 48 bases; or
(iii) the second coordinate zipcode comprises no more than 32 bases.

14. The zipcode array of claim 11, wherein:
(i) the first location is no more than 5 µm in length, optionally wherein the first location is no more than 2 µm in length; or
(ii) the zipcode array further comprises more than 1 million locations including the first and second locations, wherein each location of the more than 1 million locations is distinguishable from another; or
(iii) the first bottom adaptor is a sequencing adaptor; or
(iv) the first top adaptor is a primer.
